# EUROPEAN PATENT APPLICATION

(11) **EP 4 831 301 A1**
(43) Date of publication of application: **28.10.2026**
(21) Application number: 24908130.8
(22) Date of filing: 19.12.2024
(51) Int. Cl.: C12Q 1/6886, G01N 33/574

(54) **NOVEL CANCER PREDISPOSITION GENES DERIVED FROM MENDELIAN DISEASE-ASSOCIATED GENE GROUP**

(30) Priority: 20.12.2023 KR 20230187220
(71) Applicant: Seoul National University R&DB Foundation, Seoul 08826 (KR); Fundación del Sector Público Estatal Centro Nacional de Investigaciones Oncológicas Carlos III (F.S.P. CNIO), 28029 Madrid (ES)
(72) Inventor: YOON, Sung-Soo, Seoul 03080 (KR); KOH, Youngil, Seoul 03080 (KR); SONG, Seulki, Seoul 04322 (KR); PARK, Solip, 28029 Madrid (ES)
(74) Representative: Clarke, Modet y Cía., S.L.
(86) International application number: PCT/KR2024/020749
(87) International publication number: WO 2025/135848

(57) **Abstract**

The present disclosure relates to novel cancer predisposition genes and, more specifically, to a method for providing information for cancer predisposition diagnosis, the method comprising a step of detecting pathogenic germline variants in novel cancer predisposition genes derived from a Mendelian disorder-associated gene group, and to a composition, a kit, and a gene analysis panel for cancer predisposition diagnosis, comprising an agent capable of detecting the pathogenic germline variants in the cancer predisposition genes. The novel cancer predisposition genes of the present disclosure may be very usefully employed for cancer prevention and treatment by enabling management, diagnosis, and treatment prior to cancer onset or at an early stage of cancer onset by preemptively identifying cancer risk.

## Description

### Technical Field

The present disclosure relates to a novel cancer predisposition gene (CPG), and more specifically, to a method for providing information for cancer predisposition diagnosis, the method comprising detecting a pathogenic germline variant in a novel cancer predisposition gene derived from a Mendelian disorder-associated gene group, and to a composition, a kit, and a gene analysis panel for cancer predisposition diagnosis, comprising an agent capable of detecting the pathogenic germline variant in the cancer predisposition gene.

### Background Art

Germline variants are well known to increase the risk of cancer. To date, approximately 140 cancer predisposition genes (CPGs) have been identified through various strategies, including genome-wide association studies (GWAS) (Nature, 2014, 505: 302). However, it is believed that numerous cancer predisposition-associated genes still remain unidentified, which is presumably due to the low statistical power required to detect the association between rare pathogenic germline variants and the risk of cancer development.

In addition to the challenges in characterizing CPGs, our knowledge of their functional contributions to cancer remains limited; nevertheless, CPGs are known to be closely linked to fundamental cellular processes critical to cancer cell function. For example, representative CPGs such as *BRCA1*/*2, ATM,* and *TP53* influence DNA repair processes or genomic instability, disrupt the regulation of cellular metabolism, and confer resistance to apoptosis. Furthermore, CPGs are typically monogenic, exhibit a high-penetrance phenotype in tumors, and demonstrate bi-allelic inactivation. Analyses from large-scale cancer consortium data suggest that CPGs contribute to cancer development through mechanisms including immune evasion, epigenetic reprogramming, and sustained proliferative signaling (Cancer Discov., 2022, 12: 31).

To systematically identify novel CPGs, the present inventors focused on Mendelian disorder-associated genes obtained from Online Mendelian Inheritance in Man (OMIM) (Nucleic Acids Res., 2005, 33: D514), a catalog of human genes associated with genetic disorders primarily identified through gene association studies. The Mendelian disorder-associated genes in OMIM share similarities with CPGs due to their characteristics as single-gene determinants and high-penetrance phenotypes. Furthermore, it has been reported that patients with genetic disorders associated with rare variants in OMIM genes develop secondary phenotypes in adulthood. For instance, patients harboring rare variants in the *GBA* gene, which is associated with childhood Gaucher disease, develop Parkinson's disease later in adulthood (Curr. Opin. Neurobiol., 2021, 72: 148). From these examples, the present inventors hypothesized that among Mendelian disorder-associated genes previously considered unrelated to cancer, some may also contribute to cancer development through germline variants.

Against this background, the present inventors successfully discovered novel CPGs by determining the frequency of rare germline variants of Mendelian disorder-associated genes in cancer patients, thereby completing the present disclosure.

### Disclosure

### Technical Problem

An object of the present disclosure is to provide a method for providing information for cancer predisposition diagnosis, comprising detecting a pathogenic germline variant of a Mendelian disorder-associated gene in a biological sample isolated from a subject.

Another object of the present disclosure is to provide a composition, a kit, and a gene analysis panel for cancer predisposition diagnosis, comprising an agent capable of detecting a pathogenic germline variant of a Mendelian disorder-associated gene from a biological sample isolated from a subject.

### Technical Solution

The present disclosure is described in detail below. Meanwhile, each description and embodiment disclosed in the present disclosure may also be applied to other descriptions and embodiments. In other words, all combinations of the various elements disclosed in the present disclosure fall within the scope of the present disclosure. Furthermore, the scope of the present disclosure should not be construed as being limited by the specific descriptions set forth below.

To achieve the above object, in one general aspect, there is provided a method for providing information for cancer predisposition diagnosis, comprising: detecting a pathogenic germline variant of a Mendelian disorder-associated gene in a biological sample isolated from a subject.

The present inventors sought to discover novel cancer predisposition genes (CPGs) among Mendelian disorder-associated genes. Specifically, utilizing the Online Mendelian Inheritance in Man (OMIM) database, the inventors aimed to: (1) identify genes that exhibit an enrichment of pathogenic germline variants in cancer patients compared to controls as novel CPGs, and (2) functionally classify these novel CPGs. Following the case-control analysis, various classes of the OMIM genes were defined as potential CPGs based on (1) the frequency of biallelic inactivation and (2) gene expression profiles across diverse tissues.

Known CPGs, such as *BRCA1*/*2, TP53,* and *APC,* are often involved in essential cellular processes and are thus ubiquitously and non-specifically expressed across many tissues (Nature 505, 302-308 (2014)). Furthermore, these genes often follow the "two-hit" hypothesis, in which a pathogenic germline variant occurs in one allele and "second hit" occurs somatically in the remaining allele, thereby increasing the risk of cancer development.

However, exceptions to ubiquitous tissue expression have been identified across several CPGs, and diverse mechanisms of action extending beyond the classic CPG model have been proposed (Trends in Genetics 37, 433-443 (2021)). For instance, the α-1-antitrypsin gene, *SERPINA1,* is preferentially expressed in the liver at the RNA level and in the lung, kidney, and gastrointestinal tract at the protein level (Proc Natl Acad Sci U S A 114, E10244-e10253 (2017)); nevertheless, its pathogenic variants increase susceptibility to breast cancer (Oncotarget 6, 25815-25827 (2015)). In addition, some CPGs demonstrate that a single genetic variant is sufficient to increase cancer risk. For example, second-allele inactivation in Fanconi anemia-associated genes (*FANCA, FANCC,* and *FANCG*) that play a critical role in DNA repair and are linked to a high risk of malignancies, including acute myeloid leukemia and squamous cell carcinoma (Nature Reviews Cancer 18, 168-185 (2018)), is not as pervasive as that observed in *BRCA1*/*2-*associated cancers (Cancer Res 67, 9591-9596 (2007)). Based on these observations, the present inventors hypothesized that heterozygous carriers of pathogenic germline variants could also develop cancer via mechanisms of action that extend beyond the conventional two-hit hypothesis.

The present inventors further hypothesized that rare pathogenic germline variants in OMIM genes, such as those involved in the genetic haploinsufficiency of hereditary diseases, would also elevate the risk of tumor development. To validate this hypothesis, the present inventors conducted a comprehensive analysis utilizing large-scale cancer genomic data obtained from the Pan-Cancer Analysis of Whole Genomes (PCAWG) Network, with data from the 1000 Genomes Project serving as a normal control. The findings of the present disclosure demonstrate that various OMIM genes not previously classified as CPGs harbor pathogenic germline variants that predispose individuals within the general population to oncogenesis. Furthermore, through a comprehensive investigation into the oncogenic mechanisms of the *PAH* gene, the OMIM gene exhibiting the highest frequency of pathogenic germline variants, the present inventors identified a previously unrecognized mechanism by which OMIM genes increase cancer risk through the regulation of metabolic and immune response-related pathways.

As used herein, the term "cancer predisposition" means a state in which a specific subject has a higher risk of developing cancer compared to a normal subject. Even in the absence of current clinical manifestations of cancer, a subject with a cancer predisposition possesses a higher potential, susceptibility, or risk for future oncogenesis. Furthermore, as used herein, cancer predisposition also encompasses cases in which a subject has not yet been clinically diagnosed with cancer but already harbors cancer cells. As used herein, the term "cancer predisposition gene (CPG)" refers to a gene that, when harboring a pathogenic germline variant, places a subject at an elevated risk of developing cancer in the future. To date, approximately 140 CPGs, including *BRCA1*/*2, ATM,* and *TP53,* have been characterized; however, the present inventors focused on Mendelian disorder-associated genes to identify novel CPGs.

As used herein, the term "Mendelian disorder" refers to a genetic disease that follows Mendelian inheritance patterns, wherein a single mutation in the DNA structure leads to a pathological outcome. In particular, the Mendelian disorder in the present disclosure refers to a disorder other than cancer. In other words, cancers are excluded from the Mendelian disorders referred to in the present disclosure.

As used herein, the term "Mendelian disorder-associated gene" refers to a gene in which a mutation occurs to cause a Mendelian disorder. Gene information may be obtained from the Online Mendelian Inheritance in Man (OMIM) database (https://www.omim.org/). By definition of "Mendelian disorder" in the present disclosure, genes known to cause cancer in OMIM are excluded from the category of "Mendelian disorder-associated genes. In other words, the novel CPGs of the present disclosure are "Mendelian disorder-associated genes" known to cause genetic disorders other than cancer when germline variants occur. While not previously recognized as conventional CPGs, these genes have been identified in the present disclosure as having the potential to develop cancer as a secondary phenotype. In the present disclosure, the terms "Mendelian disorder-associated gene" and "OMIM gene" are used interchangeably, and are intended to have a meaning clearly distinct from that of conventional CPGs such as *BRCA1*/*2, ATM,* and *TP53.*

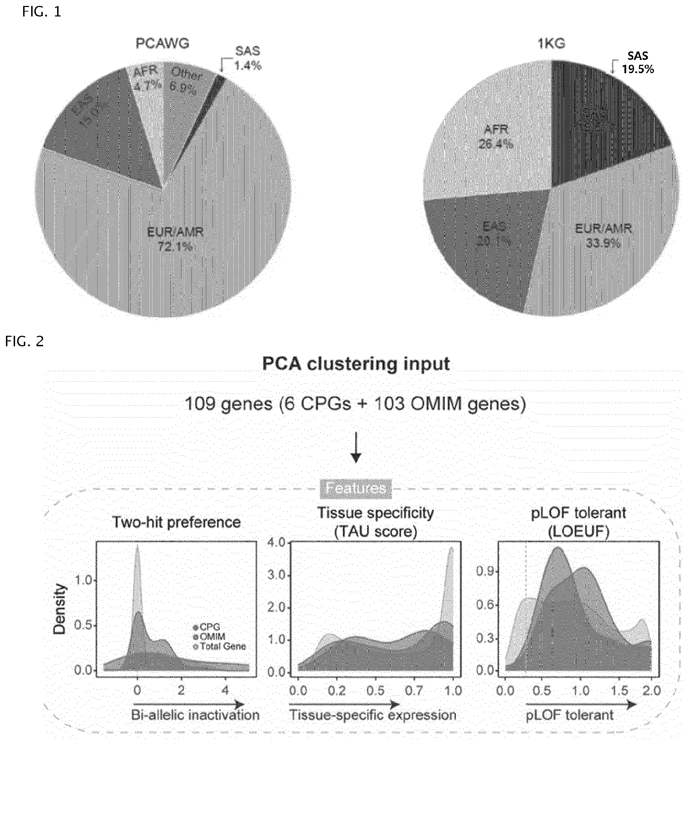

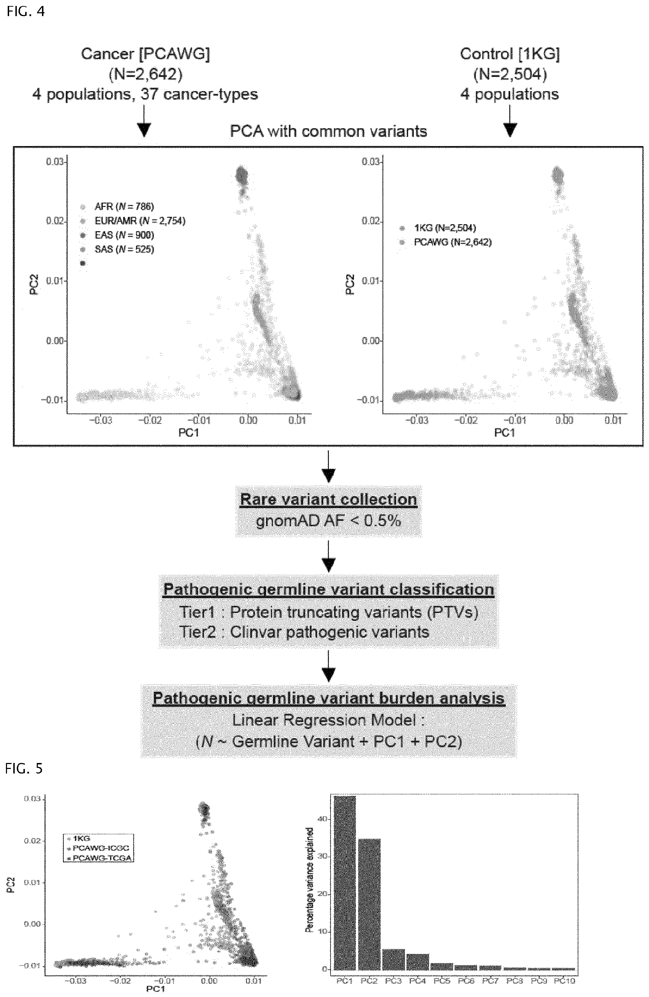

The Mendelian disorder-associated gene is one or more genes selected from the group consisting of *AAGAB, ABCA4, ABCA7, ABCC6, ABCG2, ABCG5, ACADM, ACADVL, ACPT, ADK, ADSSL1, AGBL1, AHSG, AIRE, ALB, ALPL, AMPD1, AMPD3, ANLN, ANO5, ANO6, ASB10, ASL, ASPM, ASPN, ATP5A1, ATP6AP1, ATP7B, BBS1, BCHE, BEST1, C7, C8A, C9, CALCR, CD207, CD36, CD96, CERKL, CFHR5, CFTR, CHIT1, CHST8, CLEC4M, COL9A1, CPT2, CRB1, CSF2RB, CYP1B1, CYP24A1, DAG1, DFNA5, DHCR7, DMGDH, DNAH11, DNAH17, DNAH5, DOCK6, EEF2, EPHX2, EYS, FAM111A, FCN3,* *FIG4**, FLG2, FTCD, FUT2, FYCO1, G6PC, G6PD, GABRA1, GALNS, GCKR, GJB4, GNRHR, GPNMB, GYG1, HABP2, HAVCR2, HBB, HEXB, HGD, HMCN1, HS6ST2, IFIH1, IL12RB1, IRAK3, KANK1, KEL, KIAA0586, KRT74, KRT8, LDB3, LIPH, LRP2, LRRK2, LZTR1, MAD1L1, MC1R, MEFV, MIB1, MMAA, MMP19, MMP20, MOCOS, MPO, MS4A2, MUC7, MVK, MYH8, MYO18B, MYOC, NAGA, NIPAL4, NPC2, NR1H4, OBSL1, OCA2, OGG1, OTOG, OTOGL, PADI4, PARK2, PAX4, PCCB, PCDH15, PCK2, PER3, PEX1, PHYH, PHYKPL, PKHD1, PLEKHG2, PLOD3, POLG, PREPL, PRSS12, RNASEL, RP1, SAMD9L, SI, SKIV2L, SLC12A3, SLC22A12, SLC24A4, SLC26A8, SLC3A1, SLC7A9, SLCO2A1, SLFN14, SNRPN, SPG7, SPTB, SUGCT, TBC1D8B, TCHH, TET2, TEX11, TGFBI, TGIF1, TGM5, THBD, TLR5, TMEM67, TMPRSS3, TNFRSF13B, TRPM4, TTC21A, TTN, TUBB3, TYRP1, UCP3, UPB1, USP45, VPS13B, VWA3B, WARS2,* and *WDR52.* A subject having a pathogenic germline variant in one or more genes within the above gene group may be determined to be at a high risk of developing cancer.

As used herein, the term "pathogenic germline variant" refers to a variant that results in a loss, modification, reduction, or hyperactivation of the original function of a wild-type gene due to the insertion, deletion, or substitution of one or more nucleotides or sequences therein. Specifically, the pathogenic germline variant may be a premature protein truncation variant (PTV; Tier 1) or a 'pathogenic' or 'likely pathogenic' variant according to the clinical standard ACMG/AMP criteria.

The premature protein truncation variant (PTV) refers to a variant in which the full-length sequence of a protein is not translated due to a mutation, but a truncated form of the protein is translated. For example, the PTV may be a splice donor/acceptor site variant, a frameshift indel, or a stop codon gain/loss variant, but is not limited thereto, and any variant is included within the scope of the present disclosure as long as it satisfies the definition of the pathogenic germline variant herein. For example, the pathogenic germline variant of the *PAH* gene may include, but is not limited to, F55L, I65T, N167I, R169H, V177L, V190A, Y204C, R241C, R241H, V245A, R261Q, T278I, Y356*, V388M, A403V, R408W, Y414C, or Q419R.

The "pathogenic or likely pathogenic variant according to the clinical standard ACMG/AMP criteria" encompasses all variants defined as 'pathogenic', 'likely pathogenic', 'association', or 'risk factors' based on clinical evidence developed by the American College of Medical Genetics and Genomics (ACMG) of ClinVar (https://www.ncbi.nlm.nih.gov/clinvar/), and may further encompass all potential variants that have not been identified to date but fall under the aforementioned criteria. In other words, the pathogenic germline variant in the present disclosure may be understood to mean a variant having the potential to cause a Mendelian disorder through the loss of function of a protein encoded by the corresponding gene due to a genetic mutation, and secondarily has the potential to cause cancer.

In a specific embodiment of the present disclosure, the cancer is pan-cancer, and the Mendelian disorder-associated gene may be, but is not limited to, *AAGAB, ABCG5, ACADM, ADSSL1, AGBL1, ALB, ALPL, AMPD3, ANO6, ASPN, ATP5A1, ATP6AP1, ATP7B, BEST1, C7, C8A, CD207, CD36, CD96, CSF2RB, CYP1B1, CYP24A1, DNAH11, DNAH17, DNAH5, FTCD, FUT2, FYCO1, G6PC, G6PD, HABP2, HS6ST2, IFIH1, IRAK3, KRT74, KRT8, LRRK2, MEFV, MMP19, MVK, NPC2, NR1H4, OTOG, PARK2, PAX4, PCCB, PEX1, PHYH, PHYKPL, PKHD1, PLEKHG2, PLOD3, PREPL, PRSS12, SAMD9L, SI, SLC12A3, SLC22A12, SLC26A8, SLCO2A1, SLFN14, TBC1D8B, TEX11, TGIF1, TLR5, TNFRSF13B, VWA3B* or *WDR52.*

In another specific embodiment of the present disclosure, the cancer is Biliary-AdenoCA, and the Mendelian disorder-associated gene may be, but is not limited to, *HBB.*

In another specific embodiment of the present disclosure, the cancer is Bone-Osteosarc, and the Mendelian disorder-associated gene may be, but is not limited to, *EYS.*

In another specific embodiment of the present disclosure, the cancer is Breast-AdenoCA, and the Mendelian disorder-associated gene may be, but is not limited to, *ABCA7, ACADM, AHSG, ASB10, ASPM,* C7, *DHCR7, EYS, FCN3, GCKR, LZTR1, MIB1, NAGA, PHYKPL, RNASEL, SLCO2A1, TCHH, TEX11, TMPRSS3, TTN, TYRP1, UCP3,* or *UPB1.*

In another specific embodiment of the present disclosure, the cancer is CNS-Medullo, and the Mendelian disorder-associated gene may be, but is not limited to, *CD96, CHST8, CYP24A1, DNAH17, FAM111A, GJB4, GNRHR, HABP2, HAVCR2, LRRK2, MAD1L1, MMP20, MYH8, NPC2, PRSS12, RNASEL, SAMD9L,* or *USP45.*

In another specific embodiment of the present disclosure, the cancer is CNS-PiloAstro, and the Mendelian disorder-associated gene may be, but is not limited to, C7, C9, *CHST8, SAMD9L,* or *SNRPN.*

In still another specific embodiment of the present disclosure, the cancer is ColoRect-AdenoCA, and the Mendelian disorder-associated gene may be, but is not limited to, *PCK2.*

In another specific embodiment of the present disclosure, the cancer is Eso-AdenoCA, and the Mendelian disorder-associated gene may be, but is not limited to, *ABCA7, ABCC6, ASL, GYG1, SKIV2L,* or *TTN.*

In still another specific embodiment of the present disclosure, the cancer is Head-HCC, and the Mendelian disorder-associated gene may be, but is not limited to, *FIG4**, OGG1, PHYKPL,* or *SLC7A9.*

In still another specific embodiment of the present disclosure, the cancer is Kidney-ChRCC, and the Mendelian disorder-associated gene may be, but is not limited to, *ANO5.*

In still another specific embodiment of the present disclosure, the cancer is Kidney-RCC, and the Mendelian disorder-associated gene may be, but is not limited to, *ACADM, ANLN, CD207, CHST8, CLEC4M, EEF2, FLG2, HGD, MYOC, NPC2, OTOGL, PHYKPL, SLC26A4, SUGCT, TTC21A,* or *WARS2.*

In still another specific embodiment of the present disclosure, the cancer is Liver-HCC, and the Mendelian disorder-associated gene may be, but is not limited to, *ABCA4, ABCG2, ACPT, ADK, ADSSL1, AGBL1, ALB, AMPD3, ANO5, ATP6AP1, C7, CALCR, CD207, CD36, CERKL, CFHR5, CPT2, DAG1, DMGDH, DNAH5, EYS, HABP2, HEXB, HMCN1, IL12RB1, IRAK3, KANK1, KRT74, LDB3, LIPH, LRP2, LRRK2, MEFV, MPO, MUC7, OBSL1, OTOG, PAX4, PCCB, PCDH15, PKHD1, PLOD3, PREPL, RP1, SLC22A12, SLC24A4, SLC26A8, SLFN14, SPG7, SPTB, TEX11, TGFBI,* or *VWA3B.*

In still another specific embodiment of the present disclosure, the cancer is Lung-AdenoCA, and the Mendelian disorder-associated gene may be, but is not limited to, *OCA2.*

In still another specific embodiment of the present disclosure, the cancer is Lung-SCC, and the Mendelian disorder-associated gene may be, but is not limited to, *FTCD* or *PCK2.*

In still another specific embodiment of the present disclosure, the cancer is Lymph-BNHL, and the Mendelian disorder-associated gene may be, but is not limited to, *AGBL1, C7, CFTR, GPNMB, HAVCR2, PEX1, SI, SLC3A1, TRPM4,* or *WARS2.*

In still another specific embodiment of the present disclosure, the cancer is Lymph-CLL, and the Mendelian disorder-associated gene may be, but is not limited to, ABCC6, *CFHR5, GABRA1, KIAA0586, MYO18B, RNASEL, SLC3A1,* or *TCHH.*

In still another specific embodiment of the present disclosure, the cancer is Ovary-AdenoCA, and the Mendelian disorder-associated gene may be, but is not limited to, *CFHR5, CFTR, CHST8, MAD1L1, NPC2, SI, TMEM67,* or *VPS13B.*

In still another specific embodiment of the present disclosure, the cancer is Panc-AdenoCA, and the Mendelian disorder-associated gene may be, but is not limited to, *ACADM, ACADVL, AIRE, AMPD1, BCHE, C9, CD36, CHIT1, CHST8, CRB1, CYP24A1, DFNA5, DNAH5, EPHX2, FCN3, FTCD, G6PD, LRRK2, MMAA, MMP19, MS4A2, NIPAL4, OCA2, OGG1, PADI4, POLG, SI, SUGCT, TCHH, TEX11,* or *THBD.*

In still another specific embodiment of the present disclosure, the cancer is Panc-Endocrine, and the Mendelian disorder-associated gene may be, but is not limited to, *AGBL1, PCDH15* or *SI*.

In still another specific embodiment of the present disclosure, the cancer is Prost-AdenoCA, and the Mendelian disorder-associated gene may be, but is not limited to, *CFHR5, DOCK6, G6PD, GALNS, HABP2, KRT8, MAD1L1, MMP19, MOCOS, OGG1, PER3, SLC12A3, TCHH, TET2,* or *TGM5.*

In still another specific embodiment of the present disclosure, the cancer is Skin-Melanoma, and the Mendelian disorder-associated gene may be, but is not limited to, *BBS1, DNAH11, NPC2, OCA2,* or *WARS2.*

In still another specific embodiment of the present disclosure, the cancer is Stomach-AdenoCA, and the Mendelian disorder-associated gene may be, but is not limited to, *ATP7B, COL9A1, CYP24A1, HAVCR2, IFIH1, KEL,* or *MYH8.*

In still another specific embodiment of the present disclosure, the cancer is Uterus-AdenoCA, and the Mendelian disorder-associated gene may be, but is not limited to, *GNRHR, MC1R, MPO,* or *TUBB3.*

As used herein, the term "subject" refers to a mammal, including a human. The term "biological sample" refers to any biological specimen obtained from the subject and includes, but is not limited to, blood, serum, plasma, lymph, saliva, sputum, mucus, urine, feces, or any other sample capable of detecting germline genetic variants, which is isolated from a subject to be evaluated for cancer predisposition.

In the present disclosure, the detecting of the pathogenic germline variant in the Mendelian disorder-associated gene may be performed by a method selected from the group consisting of polymerase chain reaction (PCR), Sanger sequencing, microarray, direct sequencing, next-generation sequencing, targeted exome sequencing, read-depth sequencing, and whole genome sequence assembly, but is not particularly limited thereto as long as the desired pathogenic germline variant may be detected in the target gene.

Another aspect of the present disclosure provides a composition for cancer predisposition diagnosis, comprising an agent capable of detecting a pathogenic germline variant in a Mendelian disorder-associated gene from a biological sample isolated from a subject.

The biological sample isolated from a subject and the pathogenic germline variant of the Mendelian disorder-associated gene are as described above.

The agent capable of detecting the pathogenic germline variant may be, but is not limited to, a variant-specific primer, probe, antisense nucleic acid, aptamer, or antibody.

As used herein, the term "primer" refers to a nucleic acid capable of forming base pairs with a complementary template and functioning as an initiation site for replication of the template strand. The sequence of the primer does not necessarily have to match the sequence of the template exactly, provided that it is sufficiently complementary to hybridize with the template. Primers may initiate DNA synthesis in the presence of reagents for polymerization and four different nucleoside triphosphates under appropriate buffer conditions and temperatures. PCR conditions, as well as the lengths of sense and antisense primers, may be applied and modified based on techniques well known in the art.

As used herein, the term "probe" refers to a substance capable of specifically binding to a target substance to be detected within a sample, thereby enabling the specific verification of the presence of the target substance in the sample through such binding. Probes may be constructed in forms such as, but not limited to, oligonucleotide probes, single-stranded DNA probes, double-stranded DNA probes, and RNA probes. The selection of probes and hybridization conditions may be applied and modified based on techniques well known in the art.

As used herein, the term "antisense nucleic acid" refers to a nucleic acid-based molecule that possesses a complementary nucleotide sequence to a target gene variant and is capable of forming a duplex therewith. The antisense nucleic acid may comprise the nucleotide sequence, a fragment thereof, or a sequence complementary thereto, and an appropriate length may be selected to enhance detection specificity.

In addition, the agent for detecting pathogenic germline variants according to the present disclosure may include not only agents for detecting genes but also agents capable of detecting mutant proteins translated from such genes. Examples of the agents include antibodies (e.g., monoclonal antibodies, polyclonal antibodies, chimeric antibodies, or fragments or variants thereof) or aptamers capable of specifically binding to the mutant protein, but are not limited thereto.

Another aspect of the present disclosure provides a kit for cancer predisposition diagnosis, comprising the composition.

Specifically, in addition to the composition, the kit may further comprise one or more other components, solutions, or devices suitable for the analysis method. For example, the kit may be, but is not limited to, an RT-PCR kit, a DNA chip kit, an ELISA (enzyme-linked immunosorbent assay) kit, a protein chip kit, or a rapid test kit.

Another aspect of the present disclosure provides a gene analysis panel for providing information required for cancer predisposition diagnosis, comprising the composition.

The gene analysis panel is used for a genetic variant testing method in which mutations in a plurality of target genes are configured as a single panel, and may be based on next-generation sequencing (NGS), but is not limited thereto.

The embodiments of the present disclosure may be modified into various other forms, and the scope of the present disclosure is not limited to the embodiments described below. Rather, these embodiments are provided so that this disclosure will be thorough and complete, and will fully convey the scope of the invention to those skilled in the art. Furthermore, throughout the specification, unless explicitly stated otherwise, the terms "comprise," "comprises," and "comprising" are intended to indicate the inclusion of stated elements without excluding the presence of additional elements.

### Advantageous Effects

The novel cancer predisposition genes of the present disclosure may be very usefully employed for cancer prevention and treatment by enabling management, diagnosis, and treatment prior to cancer onset or at an early stage of cancer onset by preemptively identifying cancer risk.

### Description of Drawings

FIG. 1 shows the population composition of PCAWG and 1KG (AFR: African, AMR: American, EAS: East Asian, EUR: European, and SAS: South Asian).
FIG. 2 is a schematic diagram illustrating gene classification based on four characteristics: (i) Germline enrichment: enrichment of pathogenic germline variants in patients compared to controls; (ii) Two-hit preference: an excess of germline variants in samples with LOH compared to samples without LOH; (iii) Tissue expression: the number of tissues showing high expression levels among 20 different tissues; and (iv) Tissue-specificity: the number of tissues showing high expression levels with normalized expression levels greater than 2.
FIG. 3 shows a scree plot for determining the optimal number of clusters to be used in the trimmed k-means clustering algorithm.
FIG. 4 schematically illustrates the case-control analysis. Population stratification between cancer patients and controls was adjusted by performing principal component analysis (PCA) using common variants. After collecting rare pathogenic germline variants from the case-control cohort, the enrichment of germline variants in cancer patients relative to controls was assessed using a linear regression model with the first two PC values used for population stratification.
FIG. 5 shows the population composition distribution and principal component analysis (PCA) using germline variants in PCAWG and 1KG (1000 Genomes). The PCA results according to the cohort are displayed on the left (blue: PCAWG-ICGC, green: PCAWG-TCGA, gray: 1KG). The right side shows the variance ratio for each component after PCA. The first two components account for approximately 90% of the total variance of the solution.
FIG. 6 is a graph showing genes having at least three or more carriers identified in the present disclosure, including cancer predisposition genes (CPGs), somatic driver genes (SDGs), and Online Mendelian Inheritance in Man (OMIM) genes.
FIG. 7 shows the enrichment of rare pathogenic germline variants (total: Tier 1 + 2) in cancer patients compared to controls across gene sets (** *P <* 0.01, *** *P <* 0.001).
FIG. 8 shows the log2(OR) (odds ratio) distribution from the case-control analysis across four gene sets and four subgroups of OMIM genes (* *P <* 0.05, ** *P <* 0.01, *** *P <* 0.001). The length of each whisker represents 1.5 times the interquartile range.

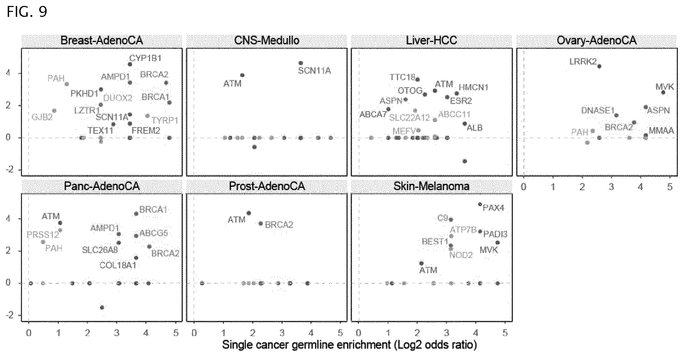

FIG. 9 shows the enrichment of pathogenic germline variants in disease classes across pan-cancer and individual cancer types using a population-adjusted linear regression model. The size of the circle indicates the degree of enrichment of pathogenic germline variants in each cancer type compared to controls, and the color indicates the disease class. Only individual cancer types with > 20 samples are displayed. Numbers in parentheses indicate the number of samples tested in each cancer type (left) and the number of OMIM genes tested in each disease (bottom). The number of diseases detected in each cancer type (P < 0.05) is displayed as a bar on the right.

### Best Mode

Hereinafter, the configuration and effects of the present disclosure will be described in further detail through Examples. These Examples are intended solely to illustrate the present disclosure, and the scope of the present disclosure is not limited by these Examples.

### Example 1: Acquisition of Tumor Sequencing Data from Pan-Cancer Analysis of Whole Genomes (PCAWG)

The final consensus set of merged germline mutation calls in the variant call format (VCF) files generated by the PCAWG consortium was obtained from the International Cancer Genome Consortium portal (https://dcc.icgc.org/releases/PCAWG). The dataset corresponded to 2,642 high-quality samples out of 2,834 donor samples, excluding 192 samples with potential technical issues (Nature, 2020, 578: 82). The germline variant calling files were divided into two germline VCF files: one containing data from 1,823 donors obtained from the non-US International Cancer Genome Consortium (ICGC), and the other containing data from 819 donors obtained from The Cancer Genome Atlas (TCGA). Each VCF file was integrated using bcftools-1.9 according to the method recommended by the ICGC data coordination center (DCC) (Bioinformatics (Oxford, England), 2011, 27: 2987). As shown in FIG. 1, the final set of PCAWG germline calls included data from 2,642 donors composed of four subgroups: European/American (1,904 donors; 72.1%), East Asian (396 donors; 15.0%), African (125 donors; 4.7%), and South Asian (36 donors; 1.4%). This also included information on 181 donors (6.9%) for whom ancestry data could not be verified. Germline variant calling was originally executed by the ICGC working group through the integration of single-nucleotide variants (SNVs), indels, and structural variations (SVs), utilizing six variant callers: GATK HaplotypeCaller (bioRxiv, 2018: 201178), FreeBayes (arXiv preprint arXiv, 2012: 1207.3907), Real Time Genomics (RTG) (bioRxiv, 2015: 023754), Delly (Bioinformatics, 2012, 28: i333), TraFiC mobile element insertion caller (https://gitlab.com/mobilegenomes/TraFiC), and Eagle2 (Nature Genetics, 2016, 48: 1443).

### Example 2: Control Exome from the 1000 Genomes Project

The exome sequences of the normal control group were collected from the 1000 Genomes Project (1KG; Phase III high-coverage whole-exome sequences) VCF files via the FTP server (ftp://ftp.1000genomes.ebi.ac.uk/vol1/ftp/release/20130502/). The dataset included germline variant information from 2,504 individuals belonging to four ancestry groups: European/American (N = 850), African (N = 661), East Asian (N = 504), and South Asian (N *=* 489) (FIG. 1). Detailed descriptions of the data generation procedures and germline variant-calling pipeline are provided in the flagship 1000 Genomes Project publication (Nature, 2015, 526: 68).

### Example 3: Clinical Information

Clinical and histopathological annotation data for PCAWG donors were obtained from the ICGC Data Portal (https://dcc.icgc.org/releases/PCAWG). The PCAWG cohort consisted of 1,462 male donors (55.3%) and 1,180 female donors (44.7%). The pan-cancer cohort included 37 tumor types, including Biliary-AdenoCA (Biliary tract adenocarcinoma), Bladder-TCC (Bladder-Transitional cell carcinoma), Bone-Benign (Bone-benign), Bone-Epith (Bone-epithelioid), Bone-Osteosarc (Bone-Sarcoma/bone), Breast-AdenoCa (Breast-Adenocarcinoma), Breast-DCIS (Breast-In situ adenocarcinoma), Breast-LobularCA (Breast-Lobular carcinoma), Cervix-AdenoCA (Cervix-Adenocarcinoma), Cervix-SCC (Cervix-Squamous cell carcinoma), CNS-GBM (Central nervous systems Diffuse glioma), CNS-Medullo (Central nervous systems Medulloblastoma), CNS-Oligo (Central nervous system Oligodendroglioma), CNS-PiloAstro (Central nervous systems Non-diffuse glioma), ColoRect-AdenoCA (Colon/Rectum Adenocarcinoma), Eso-AdenoCA (Esophagus-Adenocarcinoma), Head-SCC (Head/Neck Squamous cell carcinoma), Kidney-ChRCC (Kidney Renal cell carcinoma distal tubles), Kidney-RCC (Kidney Renal cell carcinoma), Liver-HCC (Liver-Hepatocellular carcinoma), Lung-AdenoCA (Lung-Adenocarcinoma), Lung-SCC (Lung-Squamous cell carcinoma), Lymph-BNHL (Lymphoid Mature B-cell lymphoma), Lymph-CLL (Lymphoid Chronic lymphocytic leukemia), Myeloid-AML (Myeloid-Accute myeloid leukemia), Myeloid-MDS (Myeloid Myelodysplastic syndrome), Myeloid-MPN (Myeloid-Myeloproliferative neoplasm), Ovary-AdenoCA (Ovary-Adenocarcinoma), Panc-AdenoCA (Pancreas-Adenocarcinoma), Panc-Endocrine (Pancreas-Neuroendocrine tumor), Prost-AdenoCA (Prostate-Adenocarcinoma), Skin-Melanoma (Skin-Melanoma), SoftTissue-Leiomyo (Bone/Soft tissue Sarcoma), SoftTissue-Liposarc (Bone/Soft tissue Liposarcoma), Stomach-AdenoCA (Stomach Adenocarcinoma), Thy-AdenoCA (Thyroid Adenocarcinoma), and Uterus-AdenoCA (Uterus Adenocarcinoma). Samples used for identifying germline variants primarily included not only blood-derived non-tumor samples, but also tissue samples obtained from sites adjacent to the primary tumor or other locations, including bone marrow and lymph nodes. Demographic information for the 1000 Genomes control samples is available on the 1000 Genomes portal (https://www.internationalgenome.org/data).

### Example 4: Variant Annotation and Filtering

To generate a consistent functional annotation set, each VCF file from the PCAWG (N = 2,642) and 1000 Genomes Project (1KG; N = 2,504) cohorts was edited. First, variants located within the ENCODE/DUKE (Genome Research, 2014, 24:2022) and DAC blacklist (Scientific Reports, 2019, 9:9354) regions were removed to exclude variants in low-mappability regions. Only variants located within the ENCODE/CRG GEM mappability regions (75-mers; PLOS ONE, 2012, 7:e30377) were selected. Next, the variants collected in the VCF files were annotated using ANNOVAR (version 2014 Apr 14). From the data derived via ANNOVAR's filter-based functional annotation, the following information was utilized: (i) the clinical and phenotypic effects of the variants accessed through ClinVar (accessed on June 18, 2019), and (ii) the minor allele frequency (MAF) of each variant from the Genome Aggregation Database (gnomAD) exome dataset (v2.1.1), including frequencies across eight ancestral populations - African/African American (AFR), South Asian (SAS), East Asian (EAS), Latino/Admixed American (AMR), Non-Finnish European (NFE), Finnish (FIN), Ashkenazi Jewish (ASJ), and Other (OTH) - as well as the overall population (Nature, 2020, 581:434). Simultaneously, gene-based annotations were obtained using the Ensembl Variant Effect Predictor (VEP, release 96) (Genome Biology, 2016, 17:122). From the VEP annotation data, protein-coding variants, including synonymous, missense, stop-gain, stop-loss, splice-site, frameshift indel, and in-frame indel variants, were retained for downstream analyses. Finally, variants flagged in the gnomAD exome dataset as likely technical artifacts were excluded. Specifically, variants exhibiting excess heterozygosity (InbreedingCoeff), variants with an allele count of zero after removal of low-confidence genotypes (AC0), and variants failing the random forest (RF) filtering threshold were removed from subsequent analyses.

### Example 5: Principal Component Analysis (PCA) Using Common Variants

Many germline variants identified from whole-exome sequencing data or whole genome association studies were expected to exhibit ethnicity-dependent variations. To evaluate the potential confounding effects resulting from population stratification, principal component analysis (PCA) was performed at a 90% genotyping rate using PLINK version 2.0 (Am J Hum Genet, 2007, 81: 559) on common germline variants (excluding synonymous variants) with a minor allele frequency (MAF) of ≥ 5 % across all ethnicities in the PCAWG and the 1000 Genomes Project (1KG), as well as within each ethnic group (four distinct subgroups) of PCAWG/1KG.

### Example 6: Rare Pathogenic Germline Variants

Rare variants were defined as variants having a frequency of less than 0.5% across all ethnicities in the gnomAD exome, and less than 1% in each of the eight subgroups. For variants not present in the gnomAD exome, the number of detections within the cohort (PCAWG and 1KG, evaluated separately) was assessed across all variants to eliminate potential technical artifact variants, and variant samples detected in 1% or more of the PCAWG or 1KG cohort were removed (139,678 of 1,485,345 total variants; 9.4%).

Pathogenic variants are defined from the collected rare variants as follows: (1) Tier 1 variants (potentially deleterious) were defined as premature protein-truncating variants (PTVs) comprising splice donor/acceptor site variants, frameshift indels, and stop codon gain/loss variants, which were not designated as "benign" or "likely benign" in ClinVar (http://www.ncbi.nlm.nih.gov/clinvar/; Nucleic Acids Res, 2014, 42: D980). (2) Tier 2 variants were defined as variants having a clinical significance of "pathogenic," "likely pathogenic," "associated," or "risk factor" in ClinVar, based on clinical evidence developed by the American College of Medical Genetics and Genomics (ACMG).

### Example 7: Gene Set Classification

Genes having mutations known to cause clinical diseases and phenotypes were searched from the Online Mendelian Inheritance in Man (OMIM) database (https://www.omim.org/; acquired Jan 21, 2020) through the Gene Map (genemap2.txt) and Morbid Map (mim2gene.txt). From the OMIM database, 17,076 disorder-associated genes of 5,392 diseases indicated in the Gene Map (cytogenetic location of genes) and the Morbid Map (cytogenetic location of diseases) were identified. Subsequently, for further analysis, 5,460 disorder-associated genes related with at least one phenotype (inherited disease) were extracted from the *genemap2.txt* file. The extracted genes were then cross-referenced with the genes listed in the *mim2gene.txt* file and converted to Ensembl gene names using the *annotables* package in R (https://github.com/stephenturner/annotables), thereby completing a group of 4,095 unique genes.

Next, a total of 152 known germline cancer predisposition genes (CPGs) were collected, including 114 genes from a recently published review paper (Nature, 2014, 505: 302), 11 genes from the Cancer Gene Census-Germline (http://cancer.sanger.ac.uk/census/), 12 genes through literature search (Cell, 2018, 173: 355), and 15 genes from the St. Jude PCGP germline study (New England Journal of Medicine, 2015, 373: 2336). In addition, to collect high-confidence somatic driver genes (SODs), 678 genes were first gathered from three data sources: IntOGen (Nature Reviews Cancer, 2020, 20: 555), MutSig (Nature, 2014, 505: 495), and MutPan (Nature Genetics, 2020, 52: 208). To ensure a non-redundant gene set, a CPG set (*N* = 152) was defined first, and then 3,952 OMIM genes were defined by removing 143 genes overlapping with the CPGs. Subsequently, 257 SODs were collected by removing 421 genes overlapping with either the CPGs or the OMIM genes. Thereafter, 53 CPGs, in which no clinically pathogenic variant was found in a TCGA-based germline variant analysis (Cell, 2018, 173: 355), were excluded from the final CPG set.

The final selected 4,308 genes were defined as input genes, including 99 CPGs, 207 SODs, and 3,952 OMIM genes [2,007 autosomal-recessive (AR) genes, 1,194 autosomal-dominant (AD) genes, 252 AD-AR genes (i.e., genes associated with both dominant and recessive inheritance depending on the variant), 211 X-linked genes, 53 somatic genes, 3 digenic genes, 5 isolated genes, 13 multifactorial genes, 2 Y-linked genes, and 212 genes with unknown inheritable phenotypes finally selected based on unique genetic annotations] (Table 1).

**[Table 1]**

| | | |
|---|---|---|
| CPG | | APC, ATM, ATR, BLM, BRCA1, BRCA2, BRIP1, BUB1B, CHEK2, COL7A1, DDB2, DOCK8, ERCC3, ERCC4, FAH, FANCA, FANCC, FANCG, FANCI, FANCM, GJB2, HFE, HNF1A, MEN1, MPL, MSH2, MSH6, MUTYH, NBN, NF1, PALB2, PHOX2B, PMS2, POLE, POLH, PRSS1, RAD51D, RB1, RECQL, RECQL4, RET, SDHB, SERPINA1, SH2B3, SOS1, TP53, TSHR, UROD, VHL, WRN, XPA, XPC |
| SOD | | ACO1, ACPP, AKR1C3, ALPK2, ANKRD30A, ARL16, ATG7, ATP11A, BAZ2A, BIRC3, CCDC73, CCDC91, CD1D, CDK12, CEP76, CLIP1, CNBD1, CNKSR1, COL5A3, DIS3, EIF5B, ENPEP, FAM135B, FAT1, FGFRL1, GPS2, HIST1H4E, HOXC12, HRNR, IFNAR1, IGLL5, KEAP1, KIF26B, KLHL6, KNTC1, L3MBTL3, LCTL, LILRB1, LTB, MDN1, MEF2B, MIA2, MICALCL, NFKBIE, NLRC5, NLRP5, NPFFR2, OMA1, OTUD7A, PCDHA3, PDCD2L, PDPR, PEG3, PHRF1, PJA2, PKHD1L1, POLQ, PPP6C, PRSS50, PSKH2, RBM43, RGL3, SELP, SEMA4D, SETD1B, SLC44A3, SLC4A5, SP140, SPATA31E1, SPOP, SSC5D, STK19, STRIP2, SUSD2, TCP11L2, TDRD10, TMPRSS13, TMPRSS7, TNFRSF10C, TTK, UBXN11, USP28, XIRP2, ZNF107, ZNF14, ZNF165, ZNF180, ZNF233, ZNF234, ZNF471, ZNF620, ZNF626, ZNF721, ZNF814, ZNF93 |
| OMIM | AR | AAAS, AARS2, AASS, ABCA1, ABCA12, ABCA3, ABCA4, ABCA5, ABCC2, ABCC6, ABCD4, ABCG8, ABHD5, ACAD8, ACAD9, ACADM, ACADS, ACADSB, ACADVL, ACAT1, ACE, ACOX2, ACP5, ACPT, ACY1, ADA, ADAMTS10, ADAMTS13, ADAMTS17, ADAMTS2, ADAMTSL4, ADCK3, ADCY1, ADD3, ADK, ADSL, ADSSL1, AEBP1, AGA, AGL, AGPAT2, AGRN, AGTR1, AGXT, AGXT2, AHI1, AHSG, AIMP1, AIPL1, AK7, ALDH3A2, ALDH4A1, ALDH5A1, ALDH6A1, ALDH7A1, ALDOB, ALG1, ALG12, ALG14, ALG3, ALG6, ALG9, ALKBH8, ALMS1, ALOX12B, ALOXE3, ALPK3, ALPL, ALS2, ALX3, AMACR, AMBN, AMHR2, AMPD1, AMPD3, AMT, ANGPTL3, ANKLE2, ANKS6, ANO5, ANO6, AP4B1, AP4M1, APOC2, APOPT1, APRT, APTX, ARL2BP, ARMC4, ARMC9, ARSA, ARSB, ARSG, ASAH1, ASL, ASPA, ASPH, ASPM, ASS1, ATAD1, ATF6, ATIC, ATP13A2, ATP2A1, ATP5A1, ATP6V0A2, ATP6V0A4, ATP6V1B1, ATP7B, ATP8A2, ATP8B1, AUH, AURKC, AVIL, B3GALNT2, B3GALT6, B3GALTL, B4GALT7, B9D1, BAAT, BBS1, BBS10, BBS12, BBS2, BBS7, BBS9, BCKDHA, BCKDHB, BCS1L, BDP1, BIN1, BRAT1, BSND, BTD, C10orf11, C10orf2, C12orf65, C19orf12, C1QA, C1QBP, C21orf2, C21orf59, C2CD3, C5orf42, C8A, C8B, CA12, CA2, CABP2, CABP4, CACNA2D4, CAD, CANT1, CARKD, CARS2, CASC5, CASP14, CASQ2, CATSPER1, CBS, CC2D1A, CC2D2A, CCBE1, CCDC108, CCDC11, CCDC41, CCDC8, CCT5, CD247, CD27, CD36, CD3D, CD3G, CDAN1, CDC14A, CDC45, CDC6, CDH23, CDHR1, CDK10, CDT1, CECR1, CENPE, CENPF, CENPJ, CEP135, CEP152, CEP164, CEP250, CEP290, CEP55, CERS3, CFD, CHAT, CHIT1, CHRND, CHRNE, CHRNG, CHST14, CHST8, CIITA, CIT, CKAP2L, CLDN16, CLDN19, CLEC7A, CLN3, CLN6, CLN8, CLPB, CLRN1, CNGA3, CNGB1, CNGB3, CNTN2, COA6, COASY, COG6, COL13A1, COL17A1, COL18A1, COL27A1, COL6A2, COL6A3, COLEC11, COLGALT1, COLQ, COQ6, COX10, COX15, COX412, COX6A2, CP, CPA6, CPAMD8, CPS1, CPT1A, CPT2, CR2, CRADD, CRAT, CRB1, CRB2, CRTAP, CSF2RB, CSF3R, CSPP1, CSTB, CTC1, CTDP1, CTH, CTNNA2, CTNS, CTPS1, CTSA, CTSC, CTSF, CTSK, CUBN, CUL7, CWF19L1, CYB5R3, CYP17A1, CYP1B1, CYP24A1, CYP27A1, CYP27B1, CYP2R1, CYP4F22, CYP4V2, CYP7B1, DAG1, DARS, DARS2, DBH, DBT, DCPS, DCXR, DDC, DDHD2, DDOST, DDRGK1, DDX59, DGAT1, DGUOK, DHCR24, DHCR7, DHDDS, DHODH, DHPS, DHX38, DMGDH, DMP1, DMXL2, DNAAF3, DNAH1, DNAH11, DNAH17, DNAH9, DNAI1, DNAJB2, DNASE1L3, DNMBP, DOCK6, DOK7, DONSON, DPAGT1, DPH1, DPYD, DPYS, DRC1, DSC3, DSG4, DST, DUOX2, DUOXA2, DYNC2H1, DYNC2LI1, DYSF, DZIP1L, EARS2, ECEL1, ECHS1, ECM1, EIF2AK4, EIF2B1, EIF2B2, EIF2B5, EIF3F, ELAC2, ELMOD3, ELP2, EMC1, EMP2, ENO3, EOGT, EPG5, EPHB2, EPM2A, EPRS, EPX, ERCC6, ERCC6L2, ERCC8, ESCO2, ETFA, ETFB, ETFDH, ETHE1, EXOSC2, EXOSC9, EXTL3, EYS, F13B, F7, FAM20A, FAN1, FARSB, FBP1, FBXL4, FCN3, FDXR, FECH, FGB, FGD4, FGF3, FGG, FIG4, FKBP10, FKBP14, FKRP, FKTN, FLAD1, FLG2, FLVCR2, FMO3, FOLR1, FOXRED1, FRAS1, FREM2, FSHB, FSHR, FTCD, FTO, FUCA1, FUK, FUT1, FUT2, FYCO1, G6PC, GAA, GALC, GALE, GALK1, GALNS, GALNT3, GAMT, GAS2L2, GAS8, GBA2, GBE1, GCDH, GCH1, GDAP1, GFM1, GFM2, GFPT1, GGCX, GIF, GINS1, GLB1, GLDC, GLDN, GLE1, GMPPB, GNB3, GNMT, GNPTAB, GNRHR, GOSR2, GP9, GPAA1, GPI, GPIHBP1, GPNMB, GPR179, GPR56, GPR68, GPR98, GPSM2, GRHPR, GRIP1, GRM6, GSR, GSS, GTPBP3, GUCY2D, GUF1, GUSB, GYG1, GYS2, H6PD, HADHA, HADHB, HAMP, HARS2, HAVCR2, HAX1, HEPHL1, HERC1, HEXA, HEXB, HFM1, HGD, HGF, HGSNAT, HIBCH, HINT1, HLCS, HMGCS2, HOGA1, HPCA, HPGD, HPS1, HPS3, HPSE2, HSD17B3, HSPG2, HYDIN, HYLS1, HYOU1, IARS2, IBA57, IDUA, IFNAR2, IFT140, IFT172, IFT27, IFT43, IFT57, IFT74, IFT80, IFT81, IGHMBP2, IGLL1, IKBKAP, IL10RA, IL11RA, IL12B, IL12RB1, IL17RC, IL36RN, IL7R, ILDR1, INSR, INVS, IQCB1, IQCE, IRF7, IRX5, ISG15, ISPD, ITGA7, ITGA8, ITGB2, ITGB4, ITGB6, ITPA, ITPR2, ITPR3, IVD, IYD, JAK3, KANK2, KARS, KCNJ1, KCNV2, KDSR, KERA, KHDC3L, KIAA0195, KIAA0556, KIAA0586, KIAA1109, KIAA1161, KLHL40, KLK4, KLKB1, KPTN, KREMEN1, KRT25, KRT8, KYNU, LAMA1, LAMA2, LAMA3, LAMB3, LAMC2, LAMC3, LARS, LARS2, LCAT, LDHD, LEPR, LEPRE1, LEPREL1, LGI4, LHCGR, LIAS, LIFR, LIG4, LIM2, LIMS2, LIPA, LIPE, LIPH, LIPT1, LIPT2, LMAN2L, LMBRD1, LMF1, LOXHD1, LPAR6, LPIN1, LPL, LRBA, LRIG2, LRIT3, LRP2, LRPAP1, LRRC6, LRTOMT, LSS, LTBP2, LTBP4, LYST, MAK, MAN1B1, MAN2B1, MANBA, MAPKBP1, MARS2, MARVELD2, MASP2, MC1R, MCCC1, MCCC2, MCEE, MCM3AP, MCM8, MCOLN1, MCPH1, MECR, MED23, MEGF8, MEI1, MERTK, MESP2, METTL23, MFRP, MFSD8, MGME1, MGP, MICU1, MIPEP, MKS1, MLC1, MMAA, MMAB, MMACHC, MMP1, MMP20, MMP21, MOCOS, MOCS2, MOGS, MPDZ, MPI, MPV17, MPZL2, MRE11A, MRPL44, MRPS2, MRPS22, MRPS34, MRPS7, MS4A1, MSH3, MSH5, MSRB3, MTFMT, MTHFS, MTO1, MTR, MUSK, MUT, MVK, MYO15A, MYO18B, MYO3A, MYO5B, MYO7A, MYO9A, MYSM1, NAGA, NAGS, NARS2, NBAS, NBEAL2, NCAPD2, NCAPD3, NDNL2, NDUFA12, NDUFAF3, NDUFAF4, NDUFAF5, NDUFAF6, NDUFB3, NDUFS1, NDUFS2, NDUFS7, NDUFV1, NEB, NEK8, NEK9, NEU1, NGLY1, NHLRC2, NIN, NIPAL4, NLRP7, NME8, NMNAT1, NPC1, NPC2, NPHP3, NPHP4, NPHS1, NPHS2, NR1H4, NSMCE2, NSUN2, NT5E, NUBPL, NUP107, NUP133, NUP160, NUP88, NUP93, OAT, OBSL1, OCA2, ORC1, ORC4, ORC6, OSGEP, OTOF, OTOG, OTOGL, OTUD6B, PADI3, PAH, PANK2, PAPSS2, PARK2, PARK7, PARS2, PATL2, PCBD1, PCCA, PCCB, PCDH12, PCDH15, PCK2, PCNT, PCSK1, PDE6C, PDE6G, PDSS2, PEPD, PEX1, PEX12, PEX13, PEX19, PEX2, PEX6, PEX7, PGAM2, PGAP2, PGM1, PGM3, PHKB, PHKG2, PHYH, PIBF1, PIGC, PIGG, PIGN, PIGO, PIGP, PIGQ, PIGV, PIGW, PINK1, PKD1L1, PKHD1, PLA2G5, PLA2G6, PLCZ1, PLD1, PLEC, PLEKHG2, PLEKHG5, PLG, PLOD1, PLOD2, PLOD3, PMFBP1, PMM2, PMPCA, PMPCB, PNKP, PNPLA6, PNPO, POC1B, POLG, POLR1C, POLR3A, POLR3B, POMC, POMGNT1, POMGNT2, POMT1, POMT2, POP1, POR, PPA2, PPIB, PPIP5K2, PPP1R15B, PPT1, PREPL, PRKCD, PRKRA, PROP1, PRSS12, PRSS56, PRX, PSMB8, PSMB9, PSPH, PTS, PUS1, PUS3, PVRL4, PYCR1, PYCR2, PYGL, PYGM, QARS, RAB23, RAB3GAP1, RAG1, RAG2, RAPSN, RARS, RARS2, RCBTB1, RELT, REPS1, RFT1, RINT1, RIPK1, RIPK4, RLTPR, RNASEH2A, RNASEH2B, RNASEH2C, RNPC3, RORC, RPE65, RPGRIP1, RPGRIP1L, RPIA, RRM2B, RSPH1, RSPH3, RSPO4, RTTN, SACS, SAG, SAR1B, SARDH, SBF1, SBF2, SCAPER, SCP2, SCYL1, SDCCAG8, SDR9C7, SEC23A, SEC24D, SEC31A, SECISBP2, SEPN1, SEPSECS, SERAC1, SERPINB7, SERPINB8, SGCA, SGCB, SGCD, SGCG, SGOL1, SGSH, SH3PXD2B, SHPK, SI, SIL1, SKIV2L, SLC10A2, SLC11A2, SLC12A1, SLC12A3, SLC13A5, SLC17A5, SLC19A2, SLC19A3, SLC1A1, SLC1A4, SLC22A12, SLC22A5, SLC24A1, SLC24A4, SLC24A5, SLC25A15, SLC25A20, SLC25A26, SLC25A32, SLC25A46, SLC26A1, SLC26A2, SLC26A3, SLC26A4, SLC28A1, SLC29A3, SLC2A10, SLC34A1, SLC34A2, SLC34A3, SLC35D1, SLC37A4, SLC38A8, SLC45A2, SLC46A1, SLC52A2, SLC52A3, SLC6A19, SLC6A3, SLC7A7, SLCO2A1, SLURP1, SLX4, SMARCAL1, SMPD1, SNIP1, SNX14, SOD1, SP110, SPAG1, SPATA5, SPATA7, SPEG, SPG11, SPG20, SPG21, SPINK5, SPTA1, SRD5A3, ST3GAL3, ST3GAL5, STAG3, STAMBP, STRA6, SUCLG1, SUGCT, SUN5, SURF1, SYCE1, SYNE1, SYNE4, SZT2, TAC3, TACR3, TAF6, TAT, TBC1D24, TBC1D7, TBCD, TBCE, TBCK, TBX19, TCHH, TCIRG1, TCTN1, TCTN2, TCTN3, TDO2, TDP1, TDP2, TDRD9, TEC, TELO2, TEX14, TEX15, TF, TFR2, TG, TGDS, TGM1, TGM3, TGM5, TH, THOC6, TK2, TLE6, TMCO1, TMEM126A, TMEM126B, TMEM165, TMEM216, TMEM67, TMEM70, TMEM8C, TMPRSS15, TMPRSS3, TMPRSS6, TMTC3, TNFRSF10B, TOP3A, TPO, TPP1, TPRN, TRAF3IP1, TRAPPC2L, TRAPPC6B, TRDN, TREH, TRIM32, TRIM36, TRIOBP, TRIP4, TRIT1, TRMT1, TRMT10C, TRMT5, TRMU, TRNT1, TRPM1, TRPV6, TSEN2, TSEN54, TSPEAR, TSPYL1, TTC18, TTC19, TTC21A, TTC21B, TTC37, TTC7A, TTI2, TTLL5, TTPA, TUB, TUBGCP4, TUBGCP6, TXNRD2, TYK2, TYMP, TYRP1, UBA5, UFC1, UGT1A1, UNC13D, UNC80, UPB1, UROC1, UROS, USB1, USH1C, USH1G, USH2A, USP45, UVSSA, VARS2, VIPAS39, VPS13A, VPS13B, VPS13C, VPS13D, VPS53, VWA3B, WARS2, WBP2, WDPCP, WDR19, WDR34, WDR35, WDR52, WDR66, WDR72, WDR81, WDR96, WEE2, WNT10A, WRAP53, WWOX, XDH, XRCC1, XRCC2, XRCC4, ZBTB42, ZFYVE26, ZMPSTE24, ZMYND10, ZMYND15, ZNF142, ZNF335, ZNF469, ZNHIT3, ZP1 |
| | AD | SEPTIN12, A2ML1, AAGAB, ABCA7, ABCB6, ABCC11, ABCC8, ABCG2, ADAM10, ADCY10, AGBL1, AKAP9, ANGPTL4, ANK2, ANKRD26, ANLN, ANO3, ANXA11, ANXA5, APOA1, APOA5, APOE, APOL2, APOL4, APPL1, ARHGEF10, ASPN, ASXL1, BAG3, BARD1, BCMO1, BEST1, BFSP2, BLK, BMP4, BMPR1B, BMPR2, C1S, C5, CACNA1S, CALCR, CAPN5, CARD14, CASP10, CASQ1, CASR, CD96, CDH15, CETP, CFHR5, CFI, CHI3L1, CHMP2B, CHRNA2, CHRNA4, CLCN2, COL2A1, COL4A2, COL4A3, COL9A1, CPOX, CPT1C, CRYAB, CRYBA2, CRYM, CTNNA3, CTNNB1, CTRC, CUX2, CYP2C9, DCTN1, DDX41, DDX58, DEPDC5, DFNA5, DIABLO, DIAPH3, DMPK, DNASE1, DNMT3A, DSG2, DTNA, DUSP6, ECE1, EEF2, EFHC1, EHHADH, EIF4G1, ELN, EMR2, EP300, EPHX2, ERBB4, ESR2, EYA4, F5, FAM111A, FAM111B, FAM83H, FAT2, FBLN5, FBN2, FGFR1, FGFR3, FLG, FLNB, FLT4, FOXP2, FUS, FUZ, FZD4, GABBR2, GABRA1, GATA6, GCNT2, GDF2, GDF3, GDF6, GDNF, GFAP, GHR, GJB4, GNB4, GPD2, GREM2, HABP2, HBB, HCN4, HLA-G, HMCN1, HNF1B, HNF4A, HNRNPU, HOXA11, HOXD13, HRG, HSPB1, HSPB3, IFIH1, IGF2, IL31RA, IMPG1, INS, INSL3, IRF3, IRF6, ITGA2, JAK2, KBTBD13, KCNE2, KCNH1, KCNH2, KCNQ1, KCNQ4, KCNT1, KDM6B, KIDINS220, KIF1B, KIF21A, KIF22, KIF5A, KLF1, KLHL10, KMT2D, KRIT1, KRT2, KRT4, KRT74, KRT9, LAMA4, LBR, LDB3, LDLR, LGI1, LMNA, LMNB1, LPP, LRP6, LRRK2, LZTR1, MACF1, MAP3K1, MAPK8IP3, MAPT, MBL2, MED13L, MEF2A, MEF2C, MFN2, MIB1, MLH3, MME, MMP13, MMP19, MS4A2, MST1R, MSX1, MTNR1B, MUC5B, MUC7, MVD, MYBPC3, MYH11, MYH14, MYH3, MYH6, MYH8, MYL2, MYLK2, MYOC, MYOCD, MYPN, NANOS1, NEXN, NKX2-5, NLRC4, NLRP1, NLRP12, NLRP3, NOBOX, NOD2, NOS3, NOTCH1, NOTCH3, NQO2, NR3C2, NR5A1, OPN1SW, OSBPL2, OSMR, P2RX2, P4HA2, PACS2, PALLD, PANX1, PAX2, PAX4, PCSK9, PDE11A, PDGFRB, PDYN, PER3, PIEZO2, PIKFYVE, PITX2, PKD2, PKP2, PLA2G7, PLIN1, PMP22, PMVK, POGZ, PRDM6, PRIMPOL, PRKCSH, PRLR, PRNP, PROK2, PROKR2, PRPH2, PSEN1, PSTPIP1, PTCH2, PTHLH, RAD51, RAD54L, RAX2, RBPJ, RHO, RNASEL, ROBO4, RORA, RP1L1, RTN4R, RYR1, RYR2, SAMD9L, SCN10A, SCN11A, SCN1A, SCN2A, SCN3A, SCN3B, SCN4A, SCN5A, SCN9A, SCNN1A, SCNN1B, SERPIND1, SETD2, SLC17A3, SLC17A9, SLC25A24, SLC26A8, SLC30A8, SLC39A5, SLC40A1, SLC44A4, SLC4A1, SLC52A1, SLC6A4, SLC9A3R1, SLFN14, SMAD9, SNCB, SNRPN, SNTA1, SOX2, SOX4, SOX5, SPECC1L, SPINK1, SPTB, SRGAP1, SRP72, STEAP3, SYCP3, SYNE2, TAB2, TAS2R16, TBXA2R, TCOF1, TEK, TENM4, TGFBI, TGIF1, TGM6, THBD, THPO, THRA, THRB, TINF2, TLR2, TMEM240, TMEM63A, TNC, TNFRSF1A, TNNI3, TNNI3K, TNNT2, TNPO3, TREX1, TRPA1, TRPM4, TRPV3, TRPV4, TSPAN12, TTN, TUBB1, TUBB3, TYR, UMOD, VSX1, WDR11, WFS1, WNK4, YARS, ZFPM2, ZNF644, ZNF687 |
| | AD-AR | AARS, ACD, ADRB3, AFP, AIRE, ALDH18A1, ALG8, AP4E1, APOB, AQP2, C3, CACNA1D, CAPN3, CD46, CFTR, CHRNA1, CHRNB1, CLCN1, COCH, COL11A2, COL4A4, COL6A1, CRYBB3, DCHS1, DDR2, DHTKD1, DNA2, DSC2, DSP, DSTYK, EDAR, ENAM, EPO, EVC, EVC2, F11, F12, F13A1, F2, FGA, FOXE1, FREM1, GDF1, GFI1B, GJB3, GJB6, GLRA1, GNAT1, GNE, GUCY2C, HAL, HEPACAM, HESX1, HNMT, HSPA9, HTRA1, HTT, ICK, IFNGR1, IL17RD, IMPG2, ITGA2B, ITGB3, JPH1, KIAA0196, KISS1R, KLHL3, KRT83, LIPC, LTBP3, MARS, MC4R, MEFV, METTL13, MPO, MTHFR, MYH2, NAGLU, NALCN, NEK1, NPPA, NR0B2, NTRK1, OPLAH, PDE6B, PDX1, PIEZO1, PIGT, PKLR, PLCD1, POGLUT1, POU1F1, PPP2R3C, PROC, PROM1, PRPH, PTPN22, PTPRQ, RDH12, RDH5, RELN, REN, ROM1, RP1, SAMD9, SAMHD1, SCO2, SEC23B, SERPINA6, SERPINC1, SETX, SH3TC2, SLC12A5, SLC2A9, SLC36A2, SLC3A1, SLC5A2, SLC6A20, SLC6A5, SLC7A9, SOX18, SPG7, SPR, TBX6, TBXAS1, TECTA, TICAM1, TLR3, TMC1, TNFRSF13B, UCP3, VDR, VWF, WNT10B, ZNF423 |

A total of 5,598 genes were collected as the control set from all human genes in PCAWG and the 1000 Genomes Project (1KG), excluding CPGs, SODs, OMIM genes, 196 specific disease/cancer-associated genes (https://diseases.jensenlab.org/Downloads), 100 uncharacterized proteins in the Ensembl gene descriptions annotated using the biomaRt packages in R (Nature Protocols, 2009, 4: 1184), and 126 DNA repair genes.

### Example 8: Statistical Analysis of Pathogenic Germline Variant Enrichment

A burden test analysis was performed by comparing the frequency of pathogenic germline variants within genes between cancer patients (PCAWG) and controls (1KG). This method was designed to quantify gene-level cancer risk enrichment by collapsing potential rare pathogenic germline variants using a generalized linear regression model (GLM) implemented with the stats package in R. The model was applied to each gene-tissue pair, including the pan-cancer cohort and 26 individual cancer types having more than 20 samples, as defined below:$glm \left(N∼Germline Variants+PC1+PC2, family="binomial"\right)$
wherein *N* denotes the patient group (1) or the control group (0), and *Germline Variants* denotes the number of samples harboring rare pathogenic germline variants for each gene-tissue pair.

The principal component (PC) values obtained from PCA analysis of PCAWG and 1KG against the control cohort were utilized as input values for the regression analysis. Regression coefficients and corresponding *P*-values were calculated for each gene-tissue pair using the summary function in R. A higher coefficient value denotes a stronger enrichment of pathogenic germline variants relative to the control group.

### Example 9: Enrichment of Pathogenic Germline Variants in Disease Classes and Signaling Pathways

To systematically estimate the enrichment of pathogenic germline variants in disease classes compared to the control group, 10 disease classes (cardiovascular, endocrine, epilepsy, hematologic, immunologic, hepatic, pulmonary, metabolic, muscular/skeletal, and neurological) comprising 96 heritable diseases were collected based on clinically relevant disease diagnostic data obtained from Centogene (https://www.centogene.com/diagnostics/ngspanels.html) and Blueprint Genetics (https://blueprintgenetics.com/tests/panels/). Among the 3,952 OMIM genes, 784 genes were mapped to the 10 disease classes containing 91 OMIM diseases.

A total of 186 Kyoto Encyclopedia of Genes and Genomes (KEGG) signaling pathways were collected from the Molecular Signatures Database (MSigDB version 7.0). To exclude potential redundancy among the KEGG pathways, a gene set over-representation analysis was performed by integrating network-based gene weights using Functional Link Enrichment of Gene Ontology or gene sets (LEGO V2.0). Consequently, 70 KEGG signaling pathways containing 1,721 OMIM-associated genes were collected.

Enrichment analysis was performed for each disease class or pathway according to cancer types as follows:
$glm \left(N∼Pathway or Disease classes+PC1+PC2, family="binomial"\right)$
wherein *N* denotes the patient group (1) or the control group (0), and *Pathway or Disease* classes denotes the number of samples harboring rare pathogenic germline variants for each disease class- or pathway-associated gene.

The PC values obtained from the PCA analysis of PCAWG and 1KG were utilized as input values for the regression model.

### Example 10: Acquisition of Copy-Number Alteration Data from PCAWG

Copy-number alteration (CNA) data of gene-level calls for 2,642 samples were obtained from the International Cancer Genome Consortium (ICGC) Data Portal using the files (*final_consensus_passonly.snv_mnv_indel.icgc.public.maf* and *final_consensus_passonly.snv_mnv_indel.tcga.controlled.maf*). This dataset provides a summarized intersection of genomic region calls derived from six copy-number variant callers (ABSOLUTE, ACEseq, Battenberg, cloneHD, JaBbA, and Sclust), implemented as described by Dentro et al. (Cell, 2021, 184: 2239). From the CNA data, loss of heterozygosity (LOH) is defined as a state where the minor allele copy number is 0, according to the PCAWG paper (Nature Communications, 2020, 11: 3400).

### Example 11: Two-Hit Analysis

The two-hit hypothesis was tested at the gene level across cancer types by assessing the excess of pathogenic germline variants in samples with LOH compared to samples without LOH events. The analysis was performed for each gene-tissue pair as follows:
$glm \left(N∼Germline Variants+PC1+PC2, family="binomial"\right)$
wherein N denotes a sample harboring an LOH event (1) and a sample without an LOH event (0), and *Germline Variants* denotes the number of samples harboring rare pathogenic germline variants for each gene-tissue pair.

To control for population structure within the cancer cohort, PC values obtained from the PCA analysis solely on the PCAWG samples were used as input values for the regression analysis. The regression coefficients and *P*-values for individual gene-tissue pairs were calculated using the summary function in R. A higher coefficient value indicated a stronger enrichment of pathogenic germline variants in samples with LOH compared to samples without LOH.

### Example 12: Principal Component Analysis for Gene Classification

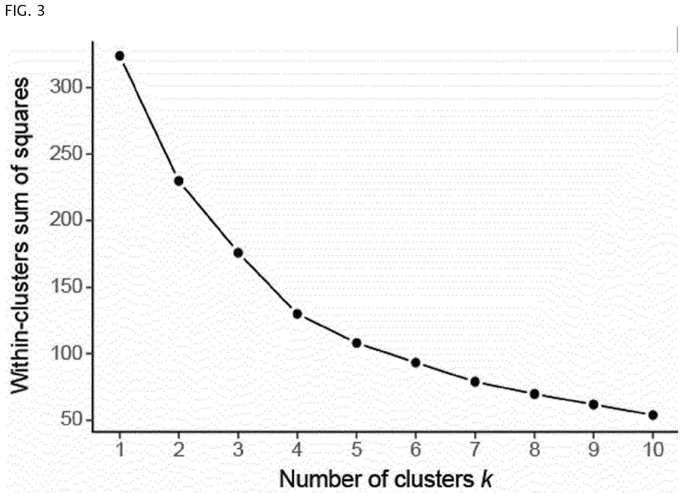

To classify OMIM genes and cancer predisposition genes (CPGs), the analysis focused on genes exhibiting 1.5-fold or higher frequency of rare germline variants in both the PCAWG and TCGA cohorts relative to the control cohort (1KG). Subsequently, four characteristics were investigated: (i) germline enrichment [prevalence of germline variants in the cancer cohort compared to normal controls (case-control analysis by a regression model; log2 odds ratio)], (ii) two-hit preference [enrichment of pathogenic germline variants in samples with LOH events compared to samples without LOH events as determined by a regression model; log2 odds ratio], (iii) tissue expression [the number of tissues exhibiting an expression level higher than the average expression value of each gene across 20 tissue types from the Human Protein Atlas (HPA)], and (iv) tissue specificity [the extent to which the expression level of a gene is broadly or specifically expressed across multiple cancer types] (FIG. 2). Tissue-specific high expression was defined as a normalized expression level (NX) greater than 2 after examining the distribution of gene expression levels across tissues in the HPA. These four values were applied as input values for a PCA-based clustering analysis using the *prcomp* function of the stats package in R. Individual input genes were classified using the top three PC values via the tclust package in R (https://cran.r-project.org/web/packages/tclust/index.html), and the classified genes were categorized into four clusters including outliers (FIG. 3).

### Example 13: Independent Cancer Cohort from TCGA

For independent validation, TCGA germline variants (PCA.r1.TCGAbarcode.merge.tnSwapCorrected.10389.vcf) for 10,389 samples were downloaded from the National Cancer Institute Genomic Data Commons (NCI GDC) legacy archive (dbGaP phs000178). The data included 7,573 Europeans (72.9%), 901 African Americans (8.7%), 651 Asians (6.3%), and 1,264 other or unidentified individuals (12.2%). For subsequent analyses, 782 samples overlapping with the PCAWG cohort were excluded, resulting in a final dataset of 9,607 TCGA samples.

### Example 14: Data Information

The present disclosure is a reanalysis of the PCAWG whole-genome sequencing (retrieved from http://dcc.icgc.org/pcawg/), the TCGA whole-exome sequencing (https://cghub.ucsc.edu/), and the 1000 Genomes Project (http://www.internationalgenome.org/). All data sets are available upon request to the research authors for the ICGC Data Access Compliance Office (DACO; http://icgc.org/daco), the TCGA Data Access Committee (DAC) via dbGaP, and the 1000 Genomes. Rare variant data sets were obtained from the gnomAD Browser version 2.1.1 (https://gnomad.broadinstitute.org/).

### Experimental Example 1: System for Measuring Enrichment of Germline Variants in Cancer

To systematically estimate whether rare pathogenic germline variants in OMIM genes contribute to cancer development, a statistical method was designed to test for the excess of rare pathogenic germline variants in cancer patients compared to normal control samples.

The approach was aimed at collecting only high-confidence pathogenic variants. Variants with a minor allele frequency (MAF) < 0.5% in the gnomAD (Genome Aggregation Database) exome were initially screened as potential pathogenic variants. Subsequently, the following three sets of potential pathogenic variants were considered through a rigorous variant curation step:
(i) Tier 1 - protein-truncating variants (PTVs), including canonical splice-site variants, frameshift indels, and nonsense variants);
(ii) Tier 2 - variants previously reported as clinically significant in the ClinVar database according to the clinical standard ACMG/AMP criteria, and classified as "Pathogenic" or "Likely Pathogenic"; and
(iii) Combined Tier 1 + 2 - a comprehensive variant set comprising both Tier 1 and Tier 2 variants to estimate the maximum contribution of potential pathogenic variants to cancer.

To determine whether the selected pathogenic variants contribute to cancer risk, single nucleotide variants (SNVs) and indels (insertions/deletions) were aggregated for each gene. A case-control analysis was utilized to identify the excess of pathogenic variants in the genes of cancer patients compared to non-cancer controls. A burden test analysis was employed to control for population structure using the first two PC values based on common variants of the case-control samples (FIG. 4 and FIG. 5).

### Experimental Example 2: Identification of Enrichment of Rare Germline Variants in Mendelian disorder-Associated Genes Across Pan-Cancer

Two data sets available with individual-level genome sequences were prepared. The first data set comprised data from multi-ancestral studies including 2,642 cancer patients across four subpopulations of the Pan-Cancer Analysis of Whole Genomes (PCAWG) Network. The second data set comprised 2,504 normal control exomes across four subpopulations of the 1000 Genomes Project (1KG) (FIG. 1). For quality control, both the PCAWG and 1KG databases included only variants in regions with sufficient sequencing coverage. A total of 7,012 pathogenic variants were identified from 2,543 genes having at least one pathogenic variant, and the frequency of pathogenic variants (Tier 1 or Tier 2) varied widely across samples and cancer types. To increase statistical power, the regression analysis was restricted to genes having at least three carriers.

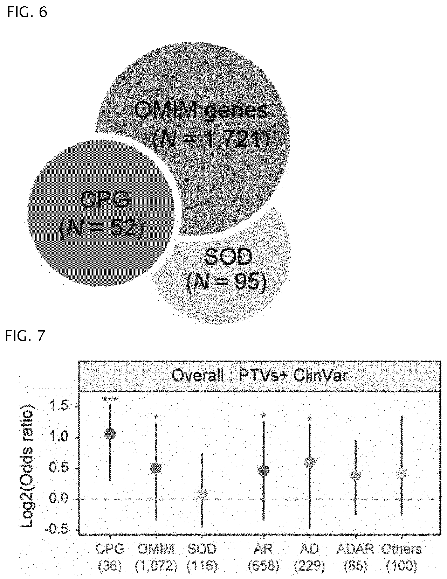

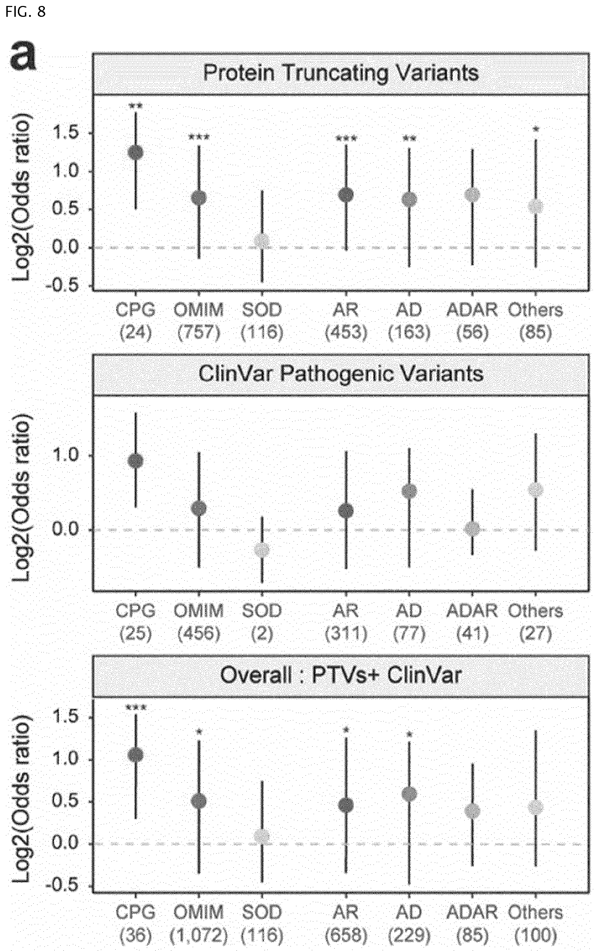

In the pan-cancer analysis, it was confirmed that known CPGs (N = 52, FIG. 6) exhibited a significant enrichment of Tier 1 variants in the cancer patient group compared to the control group (median log2 odds ratio (OR) = 1.25 for Tier 1 enrichment in cancer patients relative to controls, *P* = 1.07 × 10⁻³ by Wilcoxon rank sum test; FIG. 7 and FIG. 8). Notably, 6 CPGs (11.5%) were individually identified as exhibiting significant enrichment for Tier 1 in cancer patients relative to controls (pan-cancer patient-control analysis, P < 0.05).

Specifically, BRCA2 showed the highest cancer prevalence compared to the control group (log2(OR) = 4.21, *FDR =* 4.51 × 10⁻⁴), and additional CPGs exhibiting enrichment included *BRCA1* (log2(OR) = 3.01, *FDR =* 1.07 × 10⁻²), *FAH* (log2(OR) = 3.33, *P* = 5.33 × 10⁻²), *ATM* (log2(OR) = 2.32, *FDR* = 6.60 × 10⁻²), *BUB1B* (log2(OR) = 3.14, *P* = 8.69 × 10⁻²), and *GJB2* (log2(OR) = 1.28, *P* =8.71 × 10⁻²).

Next, it was investigated whether the OMIM Mendelian disorder-associated genes (*N* = 1,721), which are known to contribute to genetic diseases but have not previously been implicated in cancer risk, exhibit an enrichment of germline variants in cancer patients compared to control samples (FIG. 6).

The results revealed that Tier 1 variants in OMIM genes were significantly enriched in the cancer patient group relative to the control group (median log2OR = 0.65, *P* = 1.07 × 10⁻³ by Wilcoxon rank sum test, FIG. 8). This enrichment was even more pronounced when the OMIM genes were stratified into subgroups; specifically, the OMIM-autosomal recessive genes (OMIM-AR) showed a median log2(OR) = 0.69, *P* = 4.08 × 10⁻⁵ (by Wilcoxon rank sum test), while the OMIM-autosomal dominant genes (OMIM-AD) showed a median log2(OR) = 0.63, *P* = 4.08 × 10⁻⁵ (Wilcoxon rank sum test) (FIG. 8).

These findings suggest that rare pathogenic germline variants in a substantial number of OMIM genes may contribute to an increased risk of cancer.

Next, by integrating three different datasets, a set of 95 somatic driver genes (SODs), which are known to increase cancer risk through frequent somatic alterations, was used as a control gene set (FIG. 6). These genes did not exhibit an enrichment of variants in cancer (median log2(OR) = -0.29, FIG. 8).

Meanwhile, for Tier 2 variants, CPGs showed a strong enrichment in pan-cancer patients from the PCAWG (median log2(OR) = 0.99). OMIM genes also exhibited substantial enrichment (median log2(OR) = 0.31), whereas no clinically validated pathogenic variants in ClinVar were identified in the SOD gene set. Overall, Tier 1 and Tier 2 variants demonstrated similar enrichment results in pan-cancer patients. CPGs exhibited the strongest enrichment (median log2(OR) = 1.06; *P* = 4.66 × 10⁻⁴ by Wilcoxon rank sum test), followed by OMIM gene (median log2(OR) = 0.51; *P* = 1.53 × 10⁻² by Wilcoxon rank sum test) including OMIM-AR (median log2(OR) = 0.46; *P* = 1.18 × 10⁻² by Wilcoxon rank sum test) and OMIM-AD (median log2(OR) = 0.60; *P* = 4.67 × 10⁻² by Wilcoxon rank sum test).

The OMIM genes that showed statistical significance in the combined Tier 1 + 2 variant set for the pan-cancer patient cohort are presented in Table 2 below.

**[Table 2]**

| **Gene** | **log2(OR)** | ***P*-value** |
|---|---|---|
| AAGAB | 3.42 | 0.007 |
| ABCG5 | 2.35 | 0.049 |
| ACADM | 1.57 | 0.012 |
| ADSSL1 | 2.01 | 0.022 |
| AGBL1 | 0.75 | 0.045 |
| ALB | 3.51 | 0.025 |
| ALPL | 2.94 | 0.018 |
| AMPD3 | 2.15 | 0.009 |
| ANO6 | 2.64 | 0.036 |
| ASPN | 2.7 | 0.029 |
| ATP5A1 | 1.7 | 0.039 |
| ATP6AP1 | 3.42 | 0.026 |
| ATP7B | 1.39 | 0.015 |
| BEST1 | 2.23 | 0.01 |
| C7 | 1.55 | 0.006 |
| C8A | 2.58 | 0.022 |
| CD207 | 1.39 | < 0.001 |
| CD36 | 0.75 | 0.038 |
| CD96 | 3.67 | 0.017 |
| CSF2RB | 1.98 | 0.017 |
| CYP1B1 | 1.99 | 0.045 |
| CYP24A1 | 2.13 | 0.007 |
| DNAH 11 | 2.11 | 0.036 |
| DNAH17 | 2.41 | < 0.001 |
| DNAH5 | 1.43 | 0.034 |
| FTCD | 2.43 | < 0.001 |
| FUT2 | 1.74 | 0.001 |
| FYCO1 | 4.08 | 0.016 |
| G6PC | 3.34 | 0.028 |
| G6PD | 5.3 | < 0.001 |
| HABP2 | 2.07 | 0.026 |
| HS6ST2 | 3.23 | 0.03 |
| IFIH1 | 2.68 | 0.014 |
| IRAK3 | 1.95 | 0.029 |
| KRT74 | 1.61 | 0.012 |
| KRT8 | 1.5 | 0.024 |
| LRRK2 | 1.22 | 0.02 |
| MEFV | 1.87 | 0.01 |
| MMP19 | 1.57 | 0.034 |
| MVK | 3.05 | 0.044 |
| NPC2 | 1.67 | 0.012 |
| NR1H4 | 3.66 | 0.038 |
| OTOG | 1.99 | 0.002 |
| PAH | 0.88 | 0.031 |
| PARK2 | 1.71 | 0.036 |
| PAX4 | 2.86 | < 0.001 |
| PCCB | 1.91 | 0.024 |
| PEX1 | 3.46 | 0.031 |
| PHYH | 2.2 | 0.012 |
| PHYKPL | 1.35 | 0.024 |
| PKHD1 | 1.49 | 0.026 |
| PLEKHG2 | 2.87 | 0.004 |
| PLOD3 | 3.7 | 0.02 |
| PREPL | 2.18 | 0.013 |
| PRSS12 | 1.75 | 0.009 |
| SAMD9L | 1.94 | 0.033 |
| SI | 1.47 | 0.021 |
| SLC12A3 | 1.59 | 0.047 |
| SLC22A12 | 1.68 | 0.009 |
| SLC26A8 | 2.56 | 0.005 |
| SLCO2A1 | 2.69 | 0.019 |
| SLFN14 | 2.66 | < 0.001 |
| TBC1D8B | 3.33 | 0.034 |
| TEX11 | 2.3 | < 0.001 |
| TGIF1 | 2.48 | 0.043 |
| TLR5 | 1.57 | 0.044 |
| TNFRSF13B | 2.72 | 0.017 |
| VWA3B | 1.42 | 0.008 |
| WDR52 | 2.9 | 0.031 |

### Experimental Example 3: Identification of Rare Germline Variant Enrichment in OMIM Genes Across Individual Cancer Types

Next, case-control analyses were performed for each of the nine cancer types with more than 100 samples, accounting for 95.3% of all pan-cancer samples (FIG. 9).

At the single-gene level, 109 genes (6 CPGs and 103 OMIM genes) were identified to have a significant association with at least one cancer type or pan-cancer (*FDR <* 0.2).

Specifically, for CPGs, as anticipated, *BRCA1* was significantly enriched in ovarian adenocarcinoma (Ovary-AdenoCA; log2(OR) = 6.91, *FDR* = 9.63 × 10⁻⁶) and breast adenocarcinoma (Breast-AdenoCA; log2(OR) = 4.81, *P* = 4.76 × 10⁻³). Similarly, BRCA2 was significantly enriched in ovarian adenocarcinoma (Ovary-AdenoCA; log2(OR) = 4.70, *P* = 6.93 × 10⁻⁵), pancreatic adenocarcinoma (Panc-AdenoCA; log2(OR) = 4.12, *P* = 6.45 × 10⁻⁴), and breast adenocarcinoma (log2(OR) = 3.76, *P =* 9.03 × 10⁻³).

Among the OMIM genes, the following results were identified: in kidney renal cell carcinoma (Kidney-RCC), *EEF2* (log2(OR) = 5.38, FDR = 1.40 × 10⁻³) was identified as the top gene-tumor pair showing a significant level of enrichment. In addition, *PEX1* (log2(OR) = 5.13, *P* = 3.07 × 10⁻³) in B-cell non-Hodgkin lymphoma (Lymph-BNHL), *FLG2* (log2(OR) = 5.04, *P* = 3.68 × 10⁻³) in kidney renal cell carcinoma (Kidney-RCC), and *DHCR7* (log2(OR) = 4.81, *P* = 4.76 × 10⁻³) in breast adenocarcinoma (Breast-AdenoCA) also exhibited significant levels of enrichment.

The OMIM genes with significant pathogenic germline variant enrichment identified in each cancer type are summarized in Tables 3 to 25 below.

**Table 3**

| Biliary-AdenoCA | | |
|---|---|---|
| **Gene** | **log2(OR)** | ***P*-value** |
| HBB | 2.97 | < 0.001 |

**Table 4**

| Bone-Osteosarc | | |
|---|---|---|
| **Gene** | **log2(OR)** | ***P*-value** |
| EYS | 4.8 | < 0.001 |

**Table 5**

| Breast-AdenoCA | | |
|---|---|---|
| **Gene** | **log2(OR)** | ***P*-value** |
| ABCA7 | 1.64 | 0.014 |
| ACADM | 1.91 | 0.042 |
| AHSG | 2.87 | 0.007 |
| ASB10 | 1.64 | 0.043 |
| ASPM | 3.62 | 0.003 |
| C7 | 2 | 0.033 |
| DHCR7 | 4.56 | < 0.001 |
| EYS | 2.44 | 0.007 |
| FCN3 | 2.34 | 0.019 |
| GCKR | 2.73 | 0.005 |
| LZTR1 | 3.26 | 0.001 |
| MIB1 | 3.22 | 0.001 |
| NAGA | 2.47 | 0.027 |
| PHYKPL | 2.93 | 0.001 |
| RNASEL | 1.92 | 0.027 |
| SLCO2A1 | 5.13 | < 0.001 |
| TCHH | 2.15 | 0.035 |
| TEX11 | 3.46 | < 0.001 |
| TMPRSS3 | 2.16 | 0.022 |
| TTN | 1.8 | 0.018 |
| TYRP1 | 4.66 | < 0.001 |
| UCP3 | 3.01 | < 0.001 |
| UPB1 | 3.34 | 0.003 |

**Table 6**

| CNS-Medullo | | |
|---|---|---|
| **Gene** | **log2(OR)** | ***P*-value** |
| CD96 | 21.19 | < 0.001 |
| CHST8 | 3.66 | < 0.001 |
| CYP24A1 | 4.26 | 0.002 |
| DNAH17 | 4.67 | 0.009 |
| FAM111A | 21.19 | < 0.001 |
| GJB4 | 3.59 | 0.014 |
| GNRHR | 1.81 | 0.047 |
| HABP2 | 3.8 | 0.022 |
| HAVCR2 | 3.46 | 0.033 |
| LRRK2 | 3.95 | < 0.001 |
| MAD1L1 | 3.33 | 0.016 |
| MMP20 | 2.39 | 0.01 |
| MYH8 | 3.18 | 0.002 |
| NPC2 | 3.53 | 0.004 |
| PRSS12 | 4.49 | < 0.001 |
| RNASEL | 4.31 | < 0.001 |
| SAMD9L | 4.85 | 0.003 |
| USP45 | 2.19 | 0.002 |

**Table 7**

| CNS-PiloAstro | | |
|---|---|---|
| **Gene** | **log2(OR)** | ***P*-value** |
| C7 | 3.29 | 0.01 |
| C9 | 3.22 | 0.005 |
| CHST8 | 4.39 | < 0.001 |
| SAMD9L | 5.55 | 0.001 |
| SNRPN | 2.04 | 0.018 |

**Table 8**

| ColoRect-AdenoCA | | |
|---|---|---|
| **Gene** | **log2(OR)** | ***P*-value** |
| PCK2 | 2.94 | 0.001 |

**Table 9**

| Eso-AdenoCA | | |
|---|---|---|
| **Gene** | **log2(OR)** | ***P*-value** |
| ABCA7 | 3.32 | 0.034 |
| ABCC6 | 3.09 | 0.017 |
| ASL | 3.86 | 0.011 |
| GYG1 | 3.11 | 0.023 |
| SKIV2L | 5.13 | 0.006 |
| TTN | 5.48 | 0.003 |

**Table 10**

| Head-SCC | | |
|---|---|---|
| **Gene** | **log2(OR)** | ***P*-value** |
| FIG4 | 4.33 | < 0.001 |
| OGG1 | 4.71 | < 0.001 |
| PHYKPL | 3.82 | < 0.001 |
| SLC7A9 | 2.8 | 0.002 |

**Table 11**

| Kidney-ChRCC | | |
|---|---|---|
| **Gene** | **log2(OR)** | ***P*-value** |
| ANO5 | 3.94 | < 0.001 |

**Table 12**

| Kidney-RCC | | |
|---|---|---|
| **Gene** | **log2(OR)** | ***P*-value** |
| ACADM | 2.53 | 0.006 |
| ANLN | 5.15 | 0.003 |
| CD207 | 2.17 | 0.019 |
| CHST8 | 2.22 | 0.028 |
| CLEC4M | 2.23 | 0.021 |
| EEF2 | 7.43 | < 0.001 |
| FLG2 | 4.51 | < 0.001 |
| HGD | 2.13 | 0.032 |
| MYOC | 1.91 | 0.045 |
| NPC2 | 2.87 | 0.011 |
| OTOGL | 4.01 | 0.002 |
| PAH | 1.83 | 0.021 |
| PHYKPL | 2.6 | 0.011 |
| SLC26A4 | 2.93 | 0.006 |
| SUGCT | 2.47 | 0.019 |
| TTC21A | 5.88 | < 0.001 |
| WARS2 | 2.17 | 0.018 |

**Table 13**

| Liver-HCC | | |
|---|---|---|
| **Gene** | **log2(OR)** | ***P*-value** |
| ABCA4 | 2.38 | 0.013 |
| ABCG2 | 1.43 | 0.018 |
| ACPT | 5.67 | 0.006 |
| ADK | 4.06 | 0.032 |
| ADSSL1 | 2.37 | 0.045 |
| AGBL1 | 1.15 | 0.037 |
| ALB | 16.9 | < 0.001 |
| AMPD3 | 3.3 | 0.013 |
| ANO5 | 2.93 | 0.006 |
| ATP6AP1 | 16.9 | < 0.001 |
| C7 | 2.47 | 0.046 |
| CALCR | 7.21 | < 0.001 |
| CD207 | 1.96 | < 0.001 |
| CD36 | 1.35 | 0.031 |
| CERKL | 10.66 | 0.014 |
| CFHR5 | 2.74 | 0.011 |
| CPT2 | 5.4 | 0.003 |
| DAG1 | 2.57 | 0.031 |
| DMGDH | 2.59 | 0.025 |
| DNAH5 | 2.99 | 0.009 |
| EYS | 1.75 | 0.029 |
| HABP2 | 5.67 | < 0.001 |
| HEXB | 2.97 | 0.027 |
| HMCN1 | 3.25 | < 0.001 |
| IL12RB1 | 5.89 | < 0.001 |
| IRAK3 | 3.61 | 0.025 |
| KANK1 | 2.62 | 0.036 |
| KRT74 | 1.55 | 0.046 |
| LDB3 | 16.86 | < 0.001 |
| LIPH | 2.3 | 0.038 |
| LRP2 | 4.29 | 0.002 |
| LRRK2 | 1.52 | 0.039 |
| MEFV | 2.26 | 0.019 |
| MPO | 2.4 | 0.011 |
| MUC7 | 1.67 | 0.032 |
| OBSL1 | 2.8 | 0.009 |
| OTOG | 3.77 | 0.002 |
| PAX4 | 3.11 | < 0.001 |
| PCCB | 4.19 | 0.013 |
| PCDH15 | 1.79 | 0.027 |
| PKHD1 | 4.01 | 0.002 |
| PLOD3 | 16.86 | < 0.001 |
| PREPL | 2.82 | 0.014 |
| RP1 | 3 | 0.043 |
| SLC22A12 | 1.81 | 0.021 |
| SLC24A4 | 16.92 | < 0.001 |
| SLC26A8 | 10.56 | 0.021 |
| SLFN14 | 3.04 | < 0.001 |
| SPG7 | 3.65 | 0.028 |
| SPTB | 1.96 | 0.047 |
| TEX11 | 4.76 | 0.004 |
| TGFBI | 2.53 | 0.014 |
| VWA3B | 1.98 | 0.002 |

**Table 14**

| Lung-AdenoCA | | |
|---|---|---|
| **Gene** | **log2(OR)** | ***P*-value** |
| OCA2 | 3.52 | < 0.001 |

**Table 15**

| Lung-SCC | | |
|---|---|---|
| **Gene** | **log2(OR)** | ***P*-value** |
| FTCD | 8.94 | 0.001 |
| PCK2 | 2.63 | 0.007 |

**Table 16**

| Lymph-BNHL | | |
|---|---|---|
| **Gene** | **log2(OR)** | ***P*-value** |
| AGBL1 | 1.51 | 0.048 |
| C7 | 3.28 | 0.014 |
| CFTR | 2.82 | 0.022 |
| GPNMB | 8.11 | < 0.001 |
| HAVCR2 | 3.42 | 0.036 |
| PAH | 2.27 | 0.026 |
| PEX1 | 43.73 | < 0.001 |
| SI | 6.37 | 0.001 |
| SLC3A1 | 4.23 | 0.01 |
| TRPM4 | 3.48 | 0.008 |
| WARS2 | 2.78 | 0.012 |

**Table 17**

| Lymph-CLL | | |
|---|---|---|
| **Gene** | **log2(OR)** | ***P*-value** |
| ABCC6 | 3.48 | 0.019 |
| CFHR5 | 4.4 | 0.015 |
| GABRA1 | 57.73 | < 0.001 |
| KIAA0586 | 2.28 | 0.031 |
| MYO18B | 3.55 | 0.008 |
| RNASEL | 3.24 | 0.049 |
| SLC3A1 | 4.09 | 0.022 |
| TCHH | 4.02 | 0.008 |

**Table 18**

| Ovary-AdenoCA | | |
|---|---|---|
| **Gene** | **log2(OR)** | ***P*-value** |
| CFHR5 | 2.46 | 0.007 |
| CFTR | 2.57 | 0.007 |
| CHST8 | 2.49 | 0.01 |
| MAD1L1 | 3.32 | 0.003 |
| NPC2 | 3.23 | 0.003 |
| PAH | 2.18 | 0.004 |
| SI | 3.77 | < 0.001 |
| TMEM67 | 3.26 | 0.003 |
| VPS13B | 5.59 | < 0.001 |

**Table 19**

| Panc-AdenoCA | | |
|---|---|---|
| **Gene** | **log2(OR)** | ***P*-value** |
| ACADM | 1.89 | 0.038 |
| ACADVL | 3.28 | 0.016 |
| AIRE | 4.69 | 0.001 |
| AMPD1 | 3.05 | 0.001 |
| BCHE | 1.98 | 0.019 |
| C9 | 1.9 | 0.048 |
| CD36 | 3 | < 0.001 |
| CHIT1 | 2.36 | 0.023 |
| CHST8 | 1.91 | 0.03 |
| CRB1 | 4.33 | < 0.001 |
| CYP24A1 | 2.88 | 0.008 |
| DFNA5 | 5.89 | < 0.001 |
| DNAH5 | 3.3 | 0.008 |
| EPHX2 | 4.73 | 0.001 |
| FCN3 | 2.63 | 0.025 |
| FTCD | 6.99 | < 0.001 |
| G6PD | 7.72 | < 0.001 |
| LRRK2 | 2.89 | 0.012 |
| MMAA | 5.46 | < 0.001 |
| MMP19 | 3.57 | 0.011 |
| MS4A2 | 3.79 | 0.002 |
| NIPAL4 | 3.43 | 0.007 |
| OCA2 | 1.62 | 0.041 |
| OGG1 | 2.66 | 0.036 |
| PADI4 | 4.16 | < 0.001 |
| POLG | 3.15 | 0.014 |
| SI | 3.27 | 0.003 |
| SUGCT | 2.11 | 0.039 |
| TCHH | 2.39 | 0.012 |
| TEX11 | 3 | 0.02 |
| THBD | 6.13 | 0.001 |

**Table 20**

| Panc-Endocrine | | |
|---|---|---|
| **Gene** | **log2(OR)** | ***P*-value** |
| AGBL1 | 1.65 | 0.039 |
| PAH | 1.94 | 0.034 |
| PCDH15 | 4.36 | < 0.001 |
| SI | 4.88 | < 0.001 |

**Table 21**

| Prost-AdenoCA | | |
|---|---|---|
| **Gene** | **log2(OR)** | ***P*-value** |
| CFHR5 | 2.39 | 0.04 |
| DOCK6 | 4.7 | 0.014 |
| G6PD | 12.58 | < 0.001 |
| GALNS | 2.25 | 0.033 |
| HABP2 | 3.25 | 0.027 |
| KRT8 | 2.33 | 0.015 |
| MAD1L1 | 2.84 | 0.018 |
| MMP19 | 5.45 | < 0.001 |
| MOCOS | 5.92 | < 0.001 |
| OGG1 | 2.98 | 0.039 |
| PAH | 1.79 | 0.013 |
| PER3 | 6.22 | < 0.001 |
| SLC12A3 | 2.62 | 0.026 |
| TCHH | 2.25 | 0.038 |
| TET2 | 2.82 | 0.035 |
| TGM5 | 2.14 | 0.032 |

**Table 22**

| Skin-Melanoma | | |
|---|---|---|
| **Gene** | **log2(OR)** | ***P*-value** |
| BBS1 | 4.49 | 0.02 |
| DNAH 11 | 66.81 | 0.035 |
| NPC2 | 4.26 | 0.013 |
| OCA2 | 4.5 | < 0.001 |
| WARS2 | 4.1 | < 0.001 |

**Table 23**

| Stomach-AdenoCA | | |
|---|---|---|
| **Gene** | **log2(OR)** | ***P*-value** |
| ATP7B | 4.56 | < 0.001 |
| COL9A1 | 11.49 | < 0.001 |
| CYP24A1 | 5.56 | < 0.001 |
| HAVCR2 | 6.37 | < 0.001 |
| IFIH1 | 7.73 | < 0.001 |
| KEL | 3.79 | < 0.001 |
| MYH8 | 2.09 | 0.013 |

**Table 24**

| Thy-AdenoCA | | |
|---|---|---|
| **Gene** | **log2(OR)** | ***P*-value** |
| PAH | 2.97 | < 0.001 |

**Table 25**

| Uterus-AdenoCA | | |
|---|---|---|
| **Gene** | **log2(OR)** | ***P*-value** |
| GNRHR | 3.44 | < 0.001 |
| MC1R | 3.19 | < 0.001 |
| MPO | 3.38 | < 0.001 |
| TUBB3 | 3.19 | < 0.001 |

From the foregoing description, it will be understood by those skilled in the art to which the present disclosure pertains that the present disclosure may be embodied in other specific forms without departing from the technical spirit or essential characteristics thereof. In this regard, the embodiments described above are to be understood in all respects as illustrative rather than restrictive. Accordingly, the scope of the present disclosure should be determined by the appended claims, rather than by the foregoing detailed description, and all modifications and variations that fall within the meaning, scope, and equivalents of the appended claims are intended to be embraced within the scope of the present disclosure.

## Claims

1. A method for providing information for cancer predisposition diagnosis, comprising:
detecting a pathogenic germline variant of a Mendelian disorder-associated gene in a biological sample isolated from a subject,
wherein the Mendelian disorder-associated gene is one or more genes selected from the group consisting of *ATP7B, CD36,* and SI.

2. The method of claim 1, wherein the cancer is hepatocellular carcinoma or pancreatic adenocarcinoma, and the Mendelian disorder-associated gene is *CD36.*

3. The method of claim 1, wherein the cancer is mature B-cell lymphoma, ovarian adenocarcinoma, pancreatic adenocarcinoma, or a pancreatic neuroendocrine tumor, and the Mendelian disorder-associated gene is SI.

4. The method of claim 1, wherein the cancer is gastric adenocarcinoma, and the Mendelian disorder-associated gene is *ATP7B.*

5. The method of claim 1, wherein the pathogenic germline variant is a premature protein truncation variant, or a pathogenic or likely pathogenic variant according to the clinical standard ACMG/AMP criteria.

6. The method of claim 5, wherein the premature protein truncation variant is a splice donor/acceptor site variant, a frameshift indel, or a stop codon gain/loss variant.

7. The method of claim 1, wherein the biological sample is blood, serum, plasma, lymph, saliva, sputum, mucus, urine, or feces.

8. The method of claim 1, wherein the detecting of the pathogenic germline variant in the Mendelian disorder-associated gene is performed by a method selected from the group consisting of polymerase chain reaction (PCR), Sanger sequencing, microarray, direct sequencing, next-generation sequencing, targeted exome sequencing, read-depth sequencing, and whole genome sequence assembly.

9. A composition for cancer predisposition diagnosis, comprising an agent capable of detecting a pathogenic germline variant in a Mendelian disorder-associated gene from a biological sample isolated from a subject,
wherein the Mendelian disorder-associated gene is one or more genes selected from the group consisting of *ATP7B, CD36,* and *SI*.

10. The composition of claim 9, wherein the agent capable of detecting the pathogenic germline variant is a variant-specific primer, probe, antisense nucleic acid, aptamer, or antibody.

11. A kit for cancer predisposition diagnosis, comprising the composition according to claim 9 or 10.

12. A gene analysis panel for providing information required for cancer predisposition diagnosis, comprising the composition according to claim 9 or 10.
